(19) Europäisches Patentamt
European Patent Office
Office européen des brevets

(11) Veröffentlichungsnummer : **0 558 445 A1**

# (12) EUROPÄISCHE PATENTANMELDUNG

(21) Anmeldenummer : **93810085.6**

(22) Anmeldetag : **11.02.93**

(51) Int. Cl.[5] : **C07D 405/14,** C07D 409/14, C07D 405/12, C07D 409/12, A01N 47/36

(30) Priorität : **20.02.92 CH 522/92**

(43) Veröffentlichungstag der Anmeldung : **01.09.93 Patentblatt 93/35**

(84) Benannte Vertragsstaaten : **AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Kühlmeyer, Reiner, Dr.**
**Burgunderstrasse 10**
**W-7817 Ihringen 2 (DE)**
Erfinder : **Töpfl, Werner, Dr.**
**Dorneckstrasse 68**
**CH-4143 Dornach (CH)**
Erfinder : **Föry, Werner, Dr.**
**Inzlingerstrasse 11**
**CH-4125 Riehen (CH)**

## (54) Sulfonylharnstoffe als Herbizide.

(57) N-Arylsulfonyl-N'-pyrimidinyl- und N'-triazinylharnstoffe der Formel I

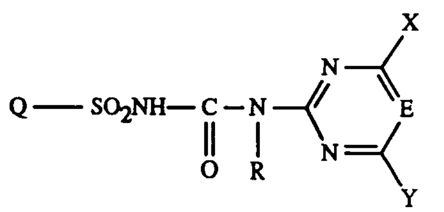

(I)

worin
Q für

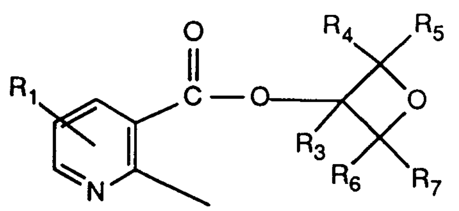

($Q_1$),

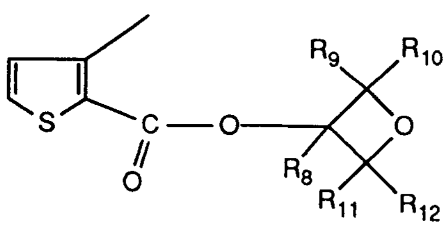

($Q_2$) oder

EP 0 558 445 A1

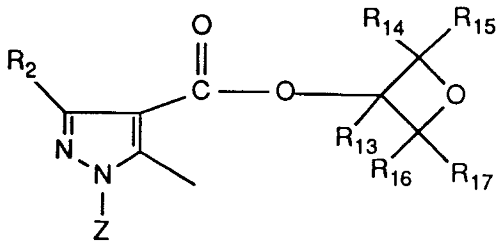

$(Q_3)$;

R für Wasserstoff oder Methyl ;
$R_1$ für Wasserstoff, Fluor, Chlor, $C_1$-$C_4$-Alkyl oder Methoxy ;
$R_2$ für Wasserstoff, Fluor oder Chlor ;
$R_2$ für Wasserstoff, Fluor oder Chlor ;
$R_3$, $R_4$, $R_5$, $R_5$ und $R_7$ unabhängig voneinander für Wasserstoff oder $C_1$-$C_4$-Alkyl ;
$R_5$, $R_9$, $R_{10}$, $R_{11}$ und $R_{12}$ unabhängig voneinander für Wasserstoff oder $C_1$-$C_4$-Alkyl ;
$R_{13}$, $R_{14}$, $R_{15}$, $R_{16}$ und $R_{17}$ unabhängig voneinander für Wasserstoff oder $C_1$-$C_4$-Alkyl ;
Z für Methyl oder 2-Pyridyl ;
E für Methin oder Stickstoff ;
X für $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, $C_2$-$C_5$-Alkoxyalkyl, $C_2$-$C_5$-Alkoxyalkoxy, $C_2$-$C_5$-Alkylthioalkyl oder Cyclopropyl ;
Y für $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino oder Di-($C_1$-$C_3$Alkyl)amino stehen ;

sowie die Salze dieser Verbindungen mit Aminen, Alkali- oder Erdalkalimetallbasen oder mit quaternären Ammoniumbasen haben gute pre- und postemergent-selektive herbizide und wuchsregulierende Eigenschaften.

Die vorliegende Erfindung betrifft neue, herbizid wirksame und pflanzenwuchsregulierende N-Arylsulfonyl-N'-pyrimidinyl- und N'-triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Harnstoffverbindungen, Triazinverbindungen und Pyrimidinverbindungen mit herbizider Wirkung sind allgemein bekannt. Derartige Verbindungen werden beispielsweise in den Europäischen Patentanmeldungen Nr. 0 007 687, 0 030 138, 0 073 562, 0 126 711 und im US-Patent Nr. 4,701,535 beschrieben.

Es wurden nun neue Sulfonylharnstoffe mit herbiziden und pflanzenwachstumsregulierenden Eigenschaften gefunden.

Die erfindungsgemäßen N-Arylsulfonyl-N'-pyrimidinyl- und N'-triazinylharnstoffe entsprechen der Formel I

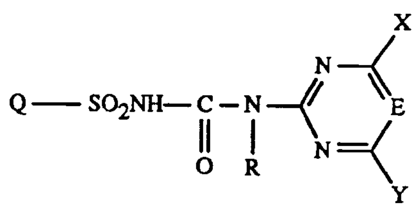

(I)

worin

Q für

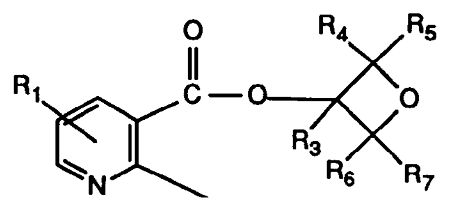

($Q_1$),

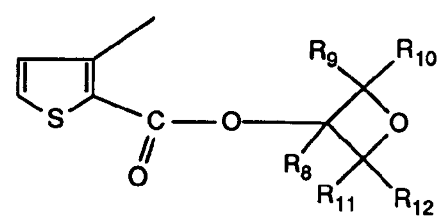

($Q_2$)

oder

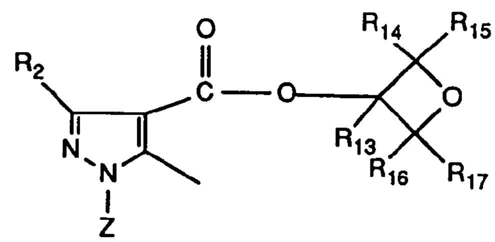

($Q_3$);

| | |
|---|---|
| R | für Wasserstoff oder Methyl; |
| $R_1$ | für Wasserstoff, Fluor, Chlor, $C_1$-$C_4$-Alkyl oder Methoxy; |
| $R_2$ | für Wasserstoff, Fluor oder Chlor; |
| $R_3$, $R_4$, $R_5$, $R_6$ und $R_7$ | unabhängig voneinander für Wasserstoff oder $C_1$-$C_4$-Alkyl; |
| $R_8$, $R_9$, $R_{10}$, $R_{11}$ und $R_{12}$ | unabhängig voneinander für Wasserstoff oder $C_1$-$C_4$-Alkyl; |
| $R_{13}$, $R_{14}$, $R_{15}$, $R_{16}$ und $R_{17}$ | unabhängig voneinander für Wasserstoff oder $C_1$-$C_4$-Alkyl; |
| Z | für Methyl oder 2-Pyridyl; |
| E | für Methin oder Stickstoff; |
| X | für $C_1$-$C_4$-Alkyl, $C_1$-$C_4$-Alkoxy, $C_1$-$C_4$-Halogenalkoxy, $C_1$-$C_4$-Halogenal- |

kylthio, $C_1$-$C_4$-Alkylthio, $C_2$-$C_5$-Alkoxyalkyl, $C_2$-$C_5$-Alkoxyalkoxy, $C_2$-$C_5$-Alkylthioalkyl oder Cyclopropyl;

Y für $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino oder Di-($C_1$-$C_3$Alkyl)amino stehen; sowie die Salze dieser Verbindungen.

Als Halogen kommt für Y in Betracht: Fluor, Chlor, Brom und Jod, vorzugsweise Fluor, Chlor und Brom.

Als $C_1$-$C_4$-Alkyl kommt für $R_1$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, X und Y in Betracht: Methyl, Ethyl, n-Propyl, iso-Propyl, n-Butyl, sek.-Butyl, iso-Butyl oder tert.-Butyl. Vorzugsweise besitzen die Alkylgruppen 1-3 Kohlenstoffatome.

Als $C_1$-$C_4$-Halogenalkyl kommt für Y insbesondere durch Fluor, Chlor, Brom oder Jod substituiertes Alkyl in Betracht. Bevorzugt sind unter diesen ein- bis dreifach durch Halogen, insbesondere Fluor oder Chlor substituierte Alkylgruppen, beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl; vorzugsweise Difluormethyl und Trifluormethyl.

Als $C_1$-$C_4$-Alkoxy kommt für X und Y beispielsweise Methoxy, Ethoxy, Propyloxy, i-Propyloxy, n-Butyloxy, iso-Butyloxy, sek.-Butyloxy und tert.-Butyloxy in Betracht; vorzugsweise Methoxy und Ethoxy.

Als $C_1$-$C_4$-Halogenalkoxy kommt für X und Y beispielsweise Difluormethoxy, Trifluormethoxy, 2,2,2-Trifluorethoxy, 1,1,2,2-Tetrafluorethoxy, 2-Fluorethoxy, 2-Chlorethoxy und 2,2-Difluorethoxy in Betracht; vorzugsweise Difluormethoxy und Trifluormethoxy.

Als $C_2$-$C_5$-Alkoxyalkyl kommt für X und Y beispielsweise Methoxymethyl, Methoxyethyl, Ethoxymethyl, Ethoxyethyl sowie Propyloxymethyl in Betracht.

Als $C_1$-$C_4$-Alkylthio kommt für X und Y z.B. in Betracht: Methylthio, Ethylthio, Propylthio, Isopropylthio, n-Butylthio, iso-Butylthio, sek.-Butylthio oder tert.-Butylthio, vorzugsweise Methylthio und Ethylthio.

Als $C_1$-$C_4$-Halogenalkylthio kommt für X und Y insbesondere durch Fluor, Chlor, Brom oder Jod substituiertes Alkylthio in Betracht. Bevorzugt sind unter diesen ein- bis dreifach durch Halogen, insbesondere Fluor oder Chlor substituierte Alkylthiogruppen, beispielsweise Fluormethylthio, Difluormethylthio, Trifluormethylthio, Chlormethylthio, Dichlormethylthio, Trichlormethylthio.

Als $C_2$-$C_5$-Alkylthioalkyl kommt für X z.B. in Betracht: Methylthioethyl, Ethylthioethyl, Propylthioethyl, Isopropylthiomethyl, vorzugsweise Methylthiomethyl und Ethylthioethyl.

Als $C_2$-$C_5$-Alkoxyalkoxy kommt z.B. für X und Y in Betracht: Methoxymethoxy, Methoxyethoxy, Methoxypropyloxy, Ethoxymethoxy, Ethoxyethoxy sowie Propyloxymethoxy.

Als $C_1$-$C_3$-Alkylamino kommt für Y beispielsweise Methylamino, Ethylamino, n-Propylamino oder iso-Propylamino in Betracht. Di($C_1$-$C_3$-alkyl)amino als Rest Y steht beispielsweise für Dimethylamino, Methylethylamino, Diethylamino oder n-Propylmethylamino.

Die Erfindung umfaßt ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Ethylamin, n-Propylamin, isopropylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-isopropylamin, Methyl-hexylamin, Methyl-nonylamin, Methyl-pentadecylamin, Methyl-octadecylamin, Ethyl-butylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-n-amylamin, Di-iso-amylamin, Dihexylamin, Diheptylamin, Dioctylamin, Ethanolamin, n-Propanolamin, iso-Propanolamin, N,N-Diethylethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Diethanolamin, Trimethylamin, Triethylamin, Tri-n-propylamin, Tri-iso-propylamin, Tri-n-butylamin, Tri-iso-butylamin, Tri-sek.-butylamin, Tri-n-amylamin; heterocyclische Amine wie z.B. Pyridin, Chinolin, iso-Chinolin, Morpholin, Piperidin, Pyrrolidin, Indolin, Chinuclidin und Azepin; primäre Arylamine wie z.B. Aniline, Methoxyaniline, Ethoxyaniline, o,m,p-Toluidine, Phenylendiamine, Benzidine, Naphthylamine und o,m,p-Chloraniline; insbesondere aber Ethyl-, Propyl-, Diethyl- oder Triethylamin, vor allem aber iso-Propylamin und Diethanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triethylbenzylammoniumkation, das Tetraethylammoniumkation, das Trimethylethylammoniumkation, aber auch das Ammoniumkation.

Unter den Verbindungen der Formel I sind diejenigen bevorzugt, in denen R Wasserstoff bedeutet, wobei vorzugsweise E für Methin oder Stickstoff steht. Unter Einschluß dieser Bevorzugungen steht vorzugsweise Q für $Q_1$, worin $R_1$ insbesondere für Wasserstoff steht, oder $Q_3$, worin $R_2$ insbesondere Wasserstoff oder Chlor bedeutet.

Ferner ist diejenige Gruppe von Verbindungen der Formel I hervorzuheben, in der Q für $Q_2$, X für $C_1$-$C_3$-Alkyl, $C_1$-$C_3$-Alkoxy, $C_1$-$C_2$-Halogenalkoxy oder Cyclopropyl; und Y für $C_1$-$C_3$-Alkyl, $C_1$-$C_3$-Alkoxy, $C_1$-$C_2$-Halogenalkoxy, Trifluormethyl, Difluormethyl, Fluromethyl, Methoxymethyl, Fluor, Chlor, Amino, Methylamino, Dimethylamino, oder Methylthio stehen, wobei E für Methin oder Stickstoff steht und R vorzugsweise Wasserstoff bedeutet.

Von den Verbindungen der Formel I sind ferner diejenigen bevorzugt, in denen
Q für $Q_1$ und $R_3$, $R_4$, $R_5$, $R_6$ und $R_7$ Wasserstoff bedeuten; oder
Q für $Q_2$ und $R_8$, $R_9$, $R_{10}$, $R_{11}$ und $R_{12}$ Wasserstoff bedeuten; oder
Q für $Q_3$ und $R_{13}$, $R_{14}$, $R_{15}$, $R_{16}$ und $R_{17}$ Wasserstoff bedeuten.

Bei besonders bevorzugten Verbindungen der Formel I stehen
X für $C_1$-$C_3$-Alkyl, $C_1$-$C_3$-Alkoxy, $C_1$-$C_2$Halogenalkoxy oder Cyclopropyl, insbesondere aber für Methyl, Methoxy, Difluormethoxy, Ethoxy oder Cyclopropyl;; und
Y für $C_1$-$C_3$-Alkyl, $C_1$-$C_3$-Alkoxy, $C_1$-$C_2$-Halogenalkoxy, Trifluormethyl, Difluormethyl, Fluromethyl, Methoxymethyl, Fluor, Chlor, Amino, Methylamino, Dimethylamino, Methylthio, insbesondere aber für Methyl, Ethyl, Methoxy, Difluormethoxy, Ethoxy, 2,2,2,-Trifluorethoxy, Chlor, Methylamino, Dimethylamino oder Methoxymethyl.

Als Einzelverbindung aus dem Umfang der Formel I ist N-[(3-oxetan-3-oxycarbonyl)-pyridin-2-yl-sulfonyl]-N'-(4,6-dimethoxypyrimidin-2-yl)-harnstoff besonders hervorzuheben.

Die Verbindungen der Formel I können hergestellt werden, indem man entweder

a) ein Sulfonamid der Formeln IIa, IIb oder IIc

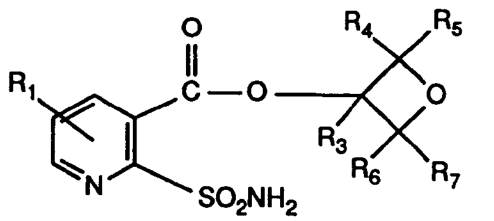

(IIa),

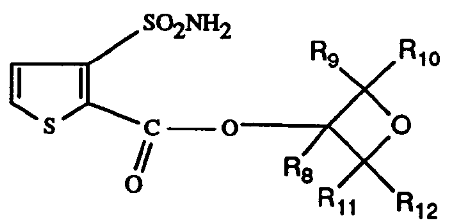

(IIb),

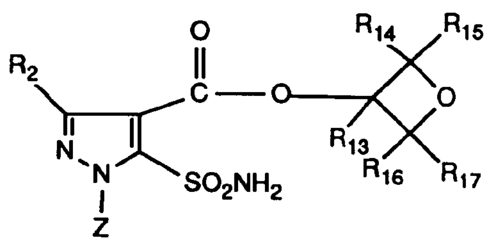

(IIc),

worin die Substituenten jeweils die unter Formel I angegebenen Bedeutungen haben, in Gegenwart einer Base mit einem Pyrimidinyl-, oder Triazinylcarbamat der Formel III

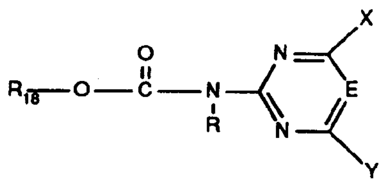

(III),

worin R, X und Y die unter Formel I angegebenen Bedeutungen haben und $R_{18}$ für Phenyl oder durch $C_1$-$C_4$-Alkyl oder Halogen substituiertes Phenyl steht, umsetzt, oder
b) ein Sulfonylcarbamat der Formel IVa, IVb, oder IVc

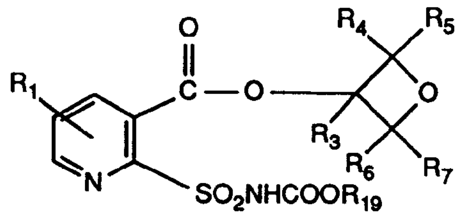

(IVa),

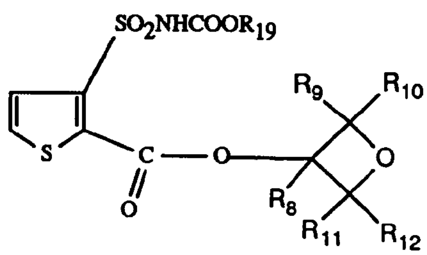

(IVb),

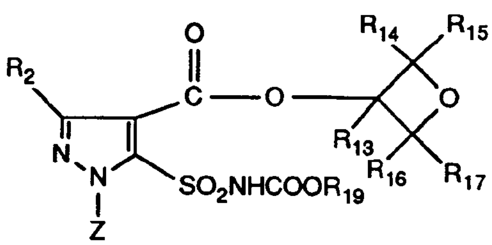

(IVc),

worin die Substituenten jeweils die unter Formel I angegebenen Bedeutungen haben und $R_{19}$ für Phenyl oder durch $C_1$-$C_4$-Alkyl oder Halogen substituiertes Phenyl steht, in Gegenwart einer Base mit einem Amin der Formel V

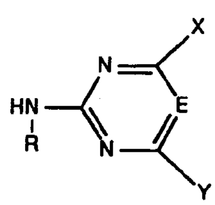

(V)

worin R, E, X und Y die unter Formel I angegebenen Bedeutungen haben, umsetzt, oder
c) ein Sulfonamid der Formeln IIa, IIb oder IIc

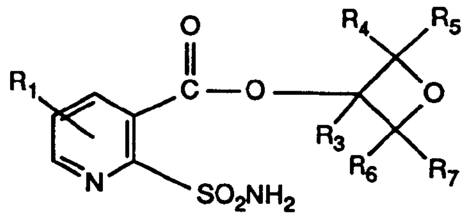

(IIa),

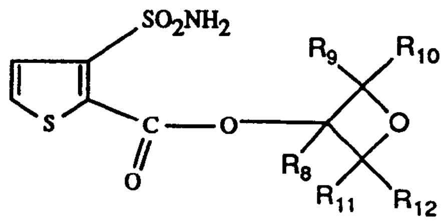

(IIb),

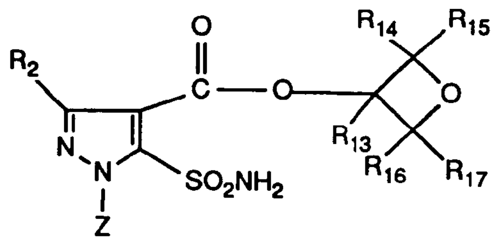

(IIc),

worin die Substituenten jeweils die unter Formel I angegebenen Bedeutungen haben, in Gegenwart einer Base mit einem Pyrimidinyl- oder Triazinylisocyanat der Formel VI

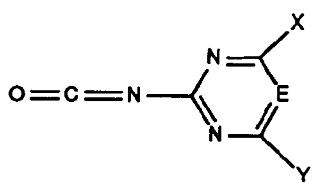

(VI)

worin E, X und Y die unter Formel I angegebenen Bedeutungen haben, umsetzt.

Verbindungen der Formel I können ferner hergestellt werden indem man eine Verbindung der Formel VIIa, VIIb oder VIIc

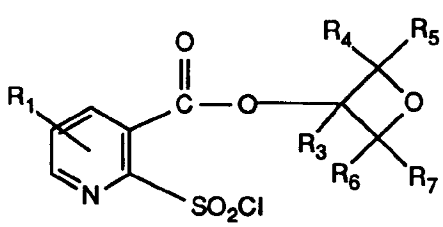

(VIIa),

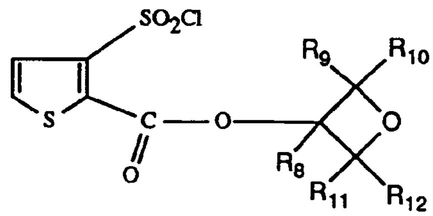

(VIIb),

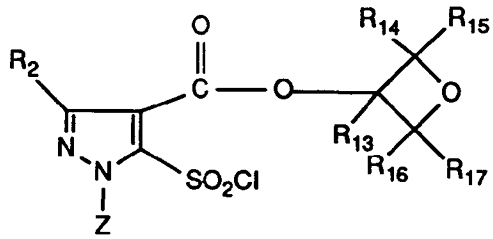

(VIIc),

worin die Substituenten jeweils die unter Formel I angegebenen Bedeutungen haben, mit einem Amin der Formel V in Gegenwart eines Ammonium-, Phosphonium-, Sulfonium- oder Alkalimetallcyanatsalzes der Formel VIII

$M^{+}OCN^{-}$ (VIII)

worin M für ein Alkalimetall oder für die Gruppe $R_{20}$, $R_{21}$, $R_{22}$, $R_{23}Q$ steht, worin $R_{20}$, $R_{21}$, $R_{22}$ und $R_{23}$ unabhängig voneinander $C_1$-$C_{18}$-Alkyl, Benzyl oder Phenyl bedeutet, wobei die Gesamtzahl der C-Atome nicht größer als 36 ist; und Q Stickstoff, Schwefel oder Phosphor bedeutet, umsetzt. Derartige Umsetzungen werden in der schweizerischen Patentschrift Nr. 662 348 beschrieben.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie Dimethylformamid, Diethylformamid oder N-Methylpyrrolidinon. Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo-(2.2.2)-octan, 1,5-Diazabicyclo(4.3.0)non-5-en oder 1,5-Diazabicyclo(5.4.0)undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffe oder chlorierten Kohlenwasserstoffe, gereinigt werden.

In den oben beschriebenen Herstellungsverfahren der Verbindungen der Formel I stehen $R_{18}$ und $R_{19}$ vorzugsweise für Phenyl, welches durch $C_1$-$C_4$-Alkyl oder Halogen substituiert sein kann, ganz besonders bevorzugt für Phenyl.

Die Sulfonamide der Formeln VIIa, VIIb und VIIc sind neue Verbindungen die speziell für die Herstellung der Wirkstoffe der Formel I entwickelt und hergestellt wurden. Sie bilden daher einen Gegenstand der vorliegenden Erfindung. Sie lassen sich aus den entsprechenden Sulfochloriden der Formel VIIa, VIIb und VIIc durch Umsetzung mit Ammoniak herstellen. Derartige Umsetzungen sind bekannt und dem Fachmann geläufig.

Die Sulfochloride der Formeln VIIa, VIIb und VIIc werden hergestellt, indem man die entsprechend substituierten 2-Chlorsulfonyl-säurechloride (siehe z.B. D.Davis, Soc. 2042, 2044 (1932)) in Gegenwart einer Base mit einer Verbindung der Formel IXa, IXb oder IXc

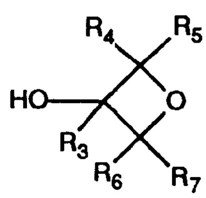

(IXa)

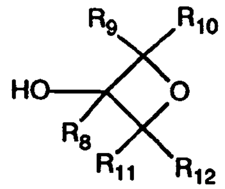

(IXb)

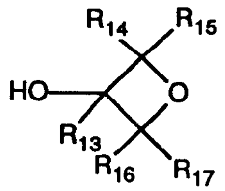

(IXc)

umsetzt. Derartige Reaktionen sind bekannt und dem Fachmann geläufig.

Phenylsulfochloride der Formeln VIIa und VIIc können auch hergestellt werden, indem man eine Verbindung der Formel Xa oder Xc

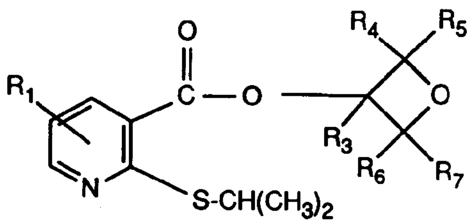

(Xa),

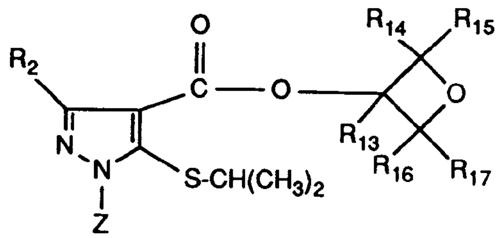

(Xc),

worin die Substituenten jeweils die unter Formel I angegebenen Bedeutungen haben, (siehe z.B. H.Gilman, F.J.Webb, Am. Soc 71, 40624063) mit Thionylchlorid zum entsprechenden Arylsäurechlorid umsetzt, welches anschließend mit den entsprechenden Verbindungen der Formeln IXa oder IXc in Gegenwart einer Base zum entsprechenden 2-Isopropylthio-arylsäureoxetan-3-ylester überführt wird um schließlich durch Umsetzung mit Chlor die Sulfochloride der Formeln VIIa und VIIc zu erhalten. Derartige Umsetzungen sind bekannt und dem Fachmann geläufig.

Verbindungen der Formel VIIb können nach folgendem, aus DE-OS 2 534 689 und 2 706 859 bekanntem Verfahren hergestellt werden:

Schema 1:

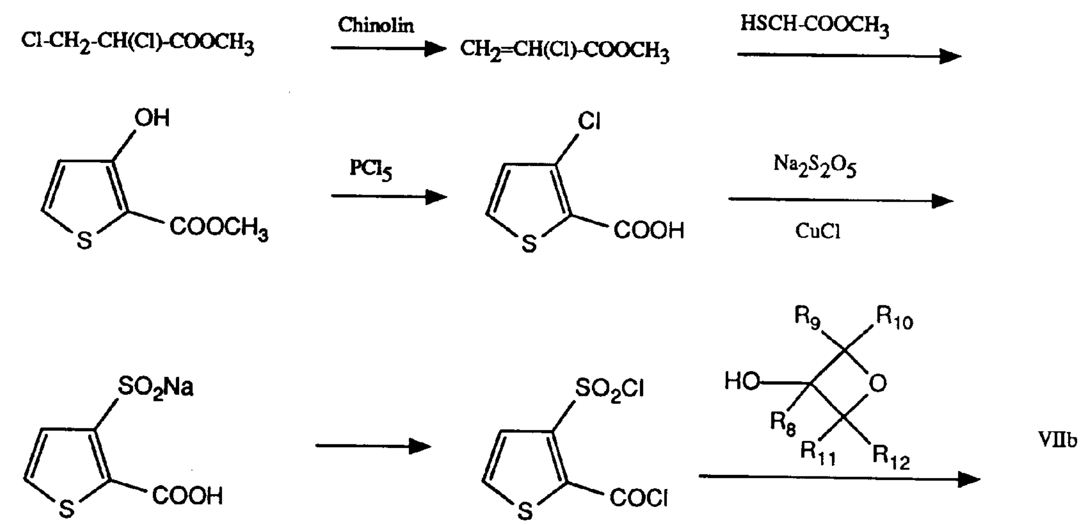


Verbindungen der Formeln IXa, IXb und IXc sind bekannt oder können analog bekannter Verfahren hergestellt werden (siehe z.B. B.Lamm et al., Acta Chem. Scand. 28, 701 (1974) oder J. Org. Chem. 48, 2953-2956 (1983)).

Die Sulfonylcarbamate der Formeln IVa, IVb und IVc können beispielsweise durch Umsetzung der Sulfonamide der Formeln IIa, IIb und IIc mit Diphenylcarbamat in Gegenwart einer Base erhalten werden. Derartige Reaktionen sind bekannt und dem Fachmann geläufig.

Die Amine der Formel V werden in den europäischen Patentanmeldungen Nr. 0 007 687, 0 030 138, 0 073 562 und 0 126 711 sowie im USP 4,579,584 beschrieben.

Verfahren zur Herstellung von N-Pyrimidinyl- und N-Triazinylcarbamaten sind beispielsweise in EP-A-0101670 beschrieben.

Die Wirkstoffe der Formel I werden in der Regel mit Aufwandmengen von 0,001 bis 2 kg/ha insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Die Verbindungen der Formel I zeichnen sich durch wuchshemmende und herbizide Eigenschaften aus, die sie ausgezeichnet zum Einsatz in Kulturen von Nutzpflanzen, insbesondere in Getreide, Baumwolle, Soja, Raps, Mais und Reis befähigen. Neben ihrer guten herbiziden Wirkung zeichnen sich die Verbindungen der Formel I auch durch ihre gute Abbaubarkeit aus.

Die Erfindung betrifft auch herbizide und pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemäßen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Straßenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkungen erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodass die Konkurrenz gegenüber der Hauptkultur ausgeschaltet ist, die agronomisch positiven Effekte wie Erosionsverhinderung, Stickstoffbindung und Bodenlockerung aber erhalten

bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Für die Verwendung der Verbindungen der Formel I oder sie enthaltender Mittel zur Regulierung des Pflanzenwachstums kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäß bis zur gleichmäßigen Verteilung auf der Samenoberfläche (Trockenbeizung). Man verwendet dabei bis zu 4 g Wirkstoff der Formel I (bei einer 50 %igen Formulierung: bis zu 8,0 g Spritzpulver) pro 1 kg Saatgut.
b) Beizung der Samen mit einem Emulsionskonzentrat des Wirkstoffs oder mit einer wäßrigen Lösung des als Spritzpulver formulierten Wirkstoffs der Formel I nach Methode a) (Naßbeizung).
c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 0,001 g bis 4,0 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formel I werden in unveränderter Form, wie aus der Synthese erhältlich, oder vorzugsweise mit den in der Formulierungstechnik üblichen Hilfsmitteln eingesetzt und werden daher z.B. zu emulgierbaren Konzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, insbesondere die Fraktionen $C_8$ bis $C_{12}$, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser; Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum , Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Disperger- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkalili-, Erdalkali oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:

- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die herbiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen: (% = Gewichtsprozent)

Emulgierbare Konzentrate:

| | |
|---|---|
| Aktiver Wirkstoff: | 1 bis 20 %, vorzugsweise 5 bis 10 % |
| oberflächenaktives Mittel: | 5 bis 30 %, vorzugsweise 10 bis 20 % |
| flüssiges Trägermittel: | 15 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspensions-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| feste Trägermittel: | 99,5 bis 70 %, vorzugsweise 97 bis 85 % |

Herstellungsbeispiele:

Beispiel H1 : Herstellung von 2-Isopropylthio-nicotinsäure

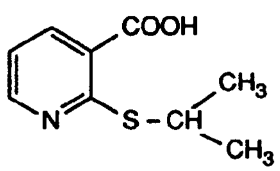

Zu einer Mischung von 50.3 g 2-Mercaptonicotinsäure und 123 ml einer 5.3 molaren methanolischen $NaOCH_3$-Lösung in 320 ml Methanol werden 33.8 ml 2-Brompropan zugetropft und unter Rückfluß erhitzt. Nach 24 Stunden wird die Suspension zur Trockne eingedampft, der Rückstand mit 250 ml Ethylacetat und 175 ml 4 N-$NH_4Cl$-Lösung bei einer Temperatur von 10°C versetzt. Sodann wird portionsweise mit 80 g $KHCO_3$ ein pH Wert von 8 eingestellt. Die wäßrige Phase wird abgetrennt mit 200 ml Ethylacetat gewaschen. Die organische Phase wird 2 mal mit je 50 ml gesättigter $KHCO_3$ Lösung extrahiert, die vereinigte wäßrige Phase mit 50 ml Eisessig versetzt und das Kristallisat abfiltriert. Nach Trocknen und Umkristallisation aus Ethylacetat/Hexan 2:1 verbleiben 47.1 g 2-Isopropylthio-nicotinsäure in Form farbloser Kristalle vom Schmelzpunkt 164-166°C.

Beispiel H2: 2-Isopropylthio-nicotinsäure-oxetan-3-yl-ester

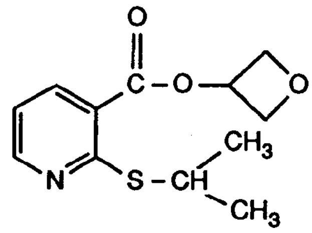

Die Mischung von 19.7 g gemäß Beispiel H1 hergestellter 2-Isopropylthionicotinsäure und 22 ml Thionylchlorid in 100 ml absolutem Toluol wird 3 Stunden bei 80°C gerührt. Nach beendeter Gasentwicklung wird filtriert und die Mutterlauge im Vakuum zur Trockne eingeengt. Das verbleibende Rohprodukt (20.8 g gelbes Öl 2-Isopropylthio-nicotinsäurechlorid) wird in 70 ml absolutem Toluol gelöst und bei einer Temperatur von 20-25°C langsam mit einer Mischung aus 8.8 g Oxetan-3-ol und 8.5 ml Pyridin gelöst in 20 ml Toluol versetzt. Nach 12-stündigem Rühren bei einer Temperatur von 20-25°C wird mit 100 ml Eiswasser sowie 200 ml Diethylether versetzt, die organische Phase abgetrennt, die wäßrige Phase 2 mal mit je 100 ml Diethylether extrahiert. Nach Trocknen und Einengen der vereinigten organischen Phase verbleiben 27.4 g 2-Isopropylthio-nicotinsäure-oxetan-3-yl-ester in Form eines hellgelben Öls. Destillative Reinigung/Badtemperatur 145-150°C, $4 \times 10^{-2}$ bar) ergeben 19.7 g Öl.

Beispiel H3: Herstellung von 3-(Oxetan-3-oxycarbonyl)-pyridin-2-yl-sulfochlorid

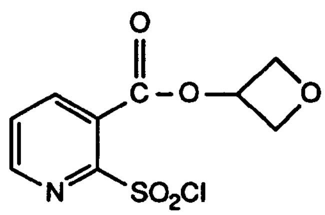

12.9 g 2-Isopropylthio-nicotinsäure-oxetan-3-ylester werden in 50 ml Wasser und 50 ml Eisessig suspendiert, mit 12.49 g Natriumacetat versetzt und unter starkem Rühren werden bei einer Temperatur von -15 bis -20°C 10.9 g Chlor eingeleitet. Die entstehende klare Lösung wird während 1 Stunde auf Raumtemperatur erwärmt, mit 300 ml Methylenchlorid und 200 ml Eiswasser versetzt. Nach Trennen der Phasen und Extrahieren der wäßrigen Phase mit 100 ml $CH_2Cl_2$ wird die vereinigte organische Phase über $MgSO_4$ getrocknet, im Vakuum eingeengt und am Hochvakuum (30°C $2 \times 10^2$ bar) getrocknet. Das so erhaltene 3-(Oxetan-3-oxycarbonyl)-pyridin-2-yl-sulfochlorid in Form eines gelben Öls (8.9 g) wird direkt zu 3-(Oxetan-3-oxycarbonyl)-pyridin-2-yl-sulfonamid umgesetzt (siehe Bsp. H4).

Beispiel H4: Herstellung von 3-(Oxetan-3-oxycarbonyl)-pyridin-2-yl-sulfonamid

(Verbindung Nr. 4.001)

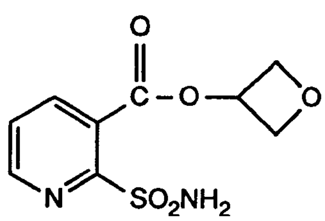

(4.001)

8.9 g 3-(Oxetan-3-oxycarbonyl)-pyridin-2-yl-sulfonchlorid werden in 150 ml absolutem $CH_2Cl_2$ gelöst und auf etwa -60°C gekühlt. Unter kräftigem Rühren werden 1.8 g $NH_3$ eingeleitet, sodann langsam auf Raumtemperatur erwärmt. Nach 1-stündigem Rühren bei Raumtemperatur wird die Suspension im Vakuum eingeengt, mit 300 ml Ethylacetat versetzt und vom Niederschlag filtriert. Nach Einengen der Mutterlauge im Vakuum verbleiben 11.8 g Rohkristallisat; nach Reinigung an Kieselgel (700 g Kieselgel, Flußmittel: Hexan/Ethylacetat 1:1)

erhält man 6.05 g 3-(Oxetan-3-oxycarbonyl)-pyridin-2-yl-sulfonamid (Verbindung Nr. 4.001) in Form weißer Kristalle vom Schmelzpunkt 140-142°C.

Beispiel H5: Herstellung von 2-(Oxetan-3-oxycarbonyl)-thiophen-3-yl-sulfonchlorid

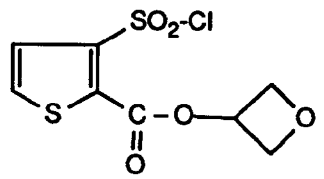

Zu einer Lösung von 14.5 g 3-Chlorsulphonyl-thiophen-2-yl-carbonsäurechlorid in 150 ml absolutem Toluol werden bei einer Temperatur von 0 bis 5°C eine Mischung von 5.2 g Oxetanol und 5 ml Pyridin in 30 ml absolutem Toluol langsam zugetropft. Nach 3 Stunden Rühren bei einer Temperatur von 0°C wird auf Raumtemperatur erwärmt, eine weitere Stunde gerührt, sodann mit 100 ml Ether und 100 ml Eiswasser versetzt. Nach Phasentrennung wird die wäßrige Phase mit 100 ml Diethylether extrahiert, die vereingte organische Phase mit 50 ml Eiswasser und 50 ml Sole gewaschen, über $Na_2SO_4$ getrocknet und im Vakuum zur Trockne eingeengt. Das verbleibende Öl wird aus einer Mischung von 25 ml Ethylacetat und 25 ml Ether umkristallisiert. Man erhält 8.7 g 2-(Oxetan-3-oxycarbonyl)-thiophen-3-yl-sulfonchlorid in Form beiger Kristalle vom Schmelzpunkt 92-94°C.

Beispiel H6: Herstellung von 2-(Oxetan-3-oxycarbonyl)-thiophen-3-yl-sulfonamid

(Verbindung 5.001):

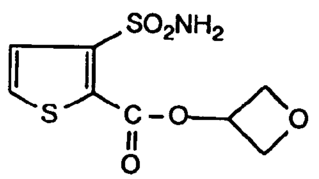

(5.001)

5.9 g 2-(Oxetan-3-oxycarbonyl)-thiophen-3-yl-sulfochlorid werden in 100 ml Methylenchlorid gelöst und auf etwa -60°C gekühlt. Unter kräftigem Rühren werden 1.8 g

Ammoniak eingeleitet, sodann langsam auf Raumtemperatur erwärmt. Nach 1-stündigem Rühren bei Raumtemperatur wird die Suspension im Vakuum eingeengt, der Rückstand mit 30 ml Wasser versetzt und 30 Minuten bei Raumtemperatur gerührt, filtriert, das Rohkristallisat mit 10 ml Methanol verrührt und anschließend filtriert. Man erhält 4.6 g 2-(Oxetan-3-oxycarbonyl)-thiophen-3-yl-sulfonamid (Verbindung 5.001) in Form eines beigen Kristallisats vom Schmelzpunkt 158-160°C.

Beispiel H7: Herstellung von 3-Chlor-4-(methyl-oxycarbonyl)-1-methyl-5-isopropyl-thio-pyrazol

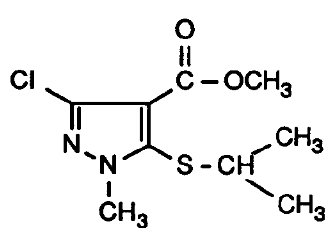

30 g 3,5-Dichlor-1-methyl-pyrazol-4-yl-carbonsäuremethylester und 25,5 g Kaliumcarbonat werden in 150 ml DMSO suspendiert und bei einer Temperatur von 25 bis 35°C langsam mit 16.1 ml Isopropylmercaptan versetzt. Nach 3-stündigem Rühren bei Raumtemperatur gibt man die Reaktionsmischung in 200 ml Eis/Wasser und versetzt anschließend mit 300 ml Ethylacetat. Nach Phasentrennung wird die organische Phase mit 100 ml Wasser sowie 100 ml gesättigte wäßrige Natriumchloridlösung gewaschen, über $MgSO_4$ getrocknet und im Vakuum zur Trockne eingeengt. Destillation des Rückstandes (113-114°C, $2 \times 10^{-3}$ bar) ergeben 31.5 g 3-Chlor-4-(methyl-oxycarbonyl)-1-methyl-5-isopropyl-thio-pyrazol in Form eines hellgelben Öls.

Beispiel H8: Herstellung von 3-Chlor-4-(oxetan-3-oxycarbonyl)-1-methyl-5-isopropylthio-pyrazol

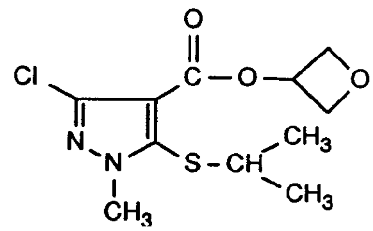

30.1 g 3-Chlor-4-(Methyl-oxycarbonyl)-1-methyl-5-isopropyl-thiopyrazol werden in 181 ml 2N NaOH suspendiert und bis zur klaren Lösung bei Raumtemperatur gerührt, sodann auf 10 bis 15°C gekühlt und mit 2N HCl auf eine pH-Wert von 2 eingestellt. Nach Filtration und Waschen mit Wasser werden 27.4 g Rohkristallisat in 150 ml Toluol suspendiert und bei einer Temperatur von 40 bis 50°C langsam mit 35 g Thionylchlorid versetzt. Nach beendeter Zugabe wird bis zur Beendigung der Gasentwicklung bei einer Temperatur von 80°C gerührt, auf Raumtemperatur gekühlt, die hellgelbe Lösung im Vakuum zur Trockne eingeengt, das verbleibende Öl (30.02 g) in 100 ml Toluol aufgenommen. Zu dieser Lösung wird das Gemisch von 16.83 g Oxetanol und 11.7 g Pyridin in 100 ml Toluol bei Raumtemperatur langsam zugetropft. Nach 10-stündigem Rühren bei Raumtemperatur gibt man die Reaktionsmischung in 250 ml Eis/Wassergemisch, trennt die Phasen und extrahiert 2 mal mit je 100 ml Ethylacetat. Die vereinigte organische Phase wird mit 100 ml Wasser und 100 ml gesättigter wässriger Natriumchloridlösung gewaschen, über $MgSO_4$ getrocknet und im Vakuum zur Trockne eingeengt. Nach Reinigung an Kieselgel (800 g Kieselgel, Flußmittel: Hexan/Ethylacetat 3:1) verbleiben 22.4 g 3-Chlor-4-(oxetan-3-oxycarbonyl)-1-methyl-5-isopropylthio-pyrazol in Form eines Öls.

Beispiel H9: Herstellung von 3-Chlor-4-(oxetan-3-oxycarbonyl)-1-methyl-pyrazol-5-yl-sulfochlorid

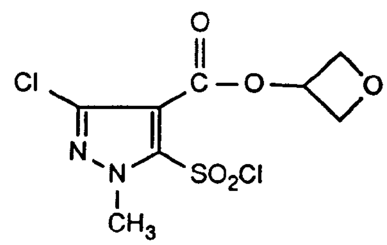

In eine Suspension von 5.8 g 3-Chlor-4-(oxetan-3-oxycarbonyl)-1-methyl-5-isopropylthiopyrazol und 5.75 g Natriumacetat in einem Gemisch aus 30 ml Eisessig und 30 ml Wasser werden bei einer Temperatur von -15 bis -20°C 4.54 g $Cl_2$ eingeleitet. Nach beendeter Zugabe erwärmt man langsam auf Raumtemperatur und versetzt mit 70 ml Methylenchlorid, sowie 30 ml Eis/Wassergemish. Nach Phasentrennung wird die wäßrige Phase mit 100 ml Methylenchlorid extrahiert, die vereinigte organische Phase mit 50 ml Wasser und 50 ml gesättigter wäßriger Natriumchloridlösung gewaschen, über $MgSO_4$ getrocknet und im Vakuum zur Trockne eingeengt; man erhält 6.26 g 3-Clor-4-(oxetan-3-oxycarbonyl)-1-methyl-pyrazol-5-yl-sulfochlorid in Form eines gelben Öls.

Beispiel H10: Herstellung von 3-Chlor-4-(oxetan-3-oxycarbonyl)-1-methyl-pyrazol-5-yl-sulfonamid

(Verbindung 6.001):

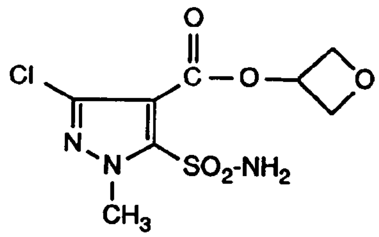

(6.001)

6.26 g 3-Chlor-4-(oxetan-3-oxycarbonyl)-1-methyl-pyrazol-5-yl-sulfochlorid werden in 50 ml THF gelöst und bei einer Temperatur von -25°C werden 1.1 g $NH_3$-Gas eingeleitet. Nach beendeter Zugabe wird langsam auf Raumtemperatur erwärmt, die Suspension filtriert und die Mutterlauge im Vakuum zur Trockne eingeengt. Das verbleibende zähe Öl wird an Kieselgel (150 g Kieselgel, Flußmittel: Hexan/Ethylacetat 1.2:1) gereinigt. Man erhält 2.1 g 3-Chlor-4-(oxetan-3-oxycarbonyl)-1-methyl-pyrazol-5-yl-sulfonamid (Verbindung 6.001) in Form farbloser Kristalle vom Schmelzpunkt 145-147°C.

Beispiel H11: Herstellung von N-[(3-oxetan-3-oxycarbonyl)-pyridin-2-yl-sulfonyl]-N'-(4,6-dimethoxypyrimidin-2-yl)-harnstoff

(Verbindung 1.001)

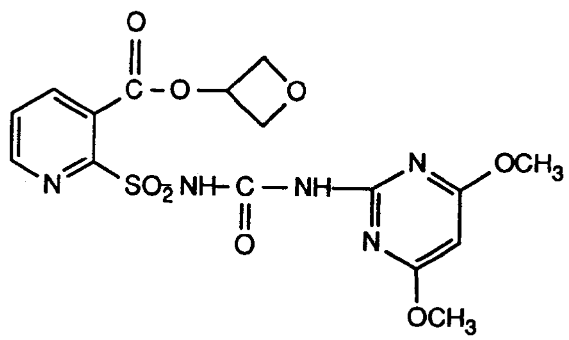

(1.001)

Eine Mischung von 1.36 g 3-(oxetan-3-oxycarbonyl)-pyridin-2-yl-sulfonamid, 1.65 g 4,6-Dimethoxy-1,3-pyrimidin-yl-phenylcarbamat und 10 ml absolutem Acetonitril werden bei einer Temperatur von 10 bis 15°C tropfenweise mit einer Mischung von 0.8 g Diazabicyclo-[5.4.0]-undec-7-en und 5 ml absolutem Acetonitril versetzt und anschließend bei Raumtemperatur 2 Stunden gerührt. Durch Eingeben in Wasser, Zutropfen von 10 %iger Salzsäure bis zur Einstellung eines pH von 5 kristallisieren 1.54 g N-[(3-oxetan-3-oxycarbonyl)-pyridin-2-yl-sulfonyl]-N'-(4,6-dimethoxypyrimidin-2-yl)-harnstoff (Verbindung 1.001) mit einem Schmelzpunkt von 175-176°C.

Tabelle 1: Verbindungen der Formel Ia

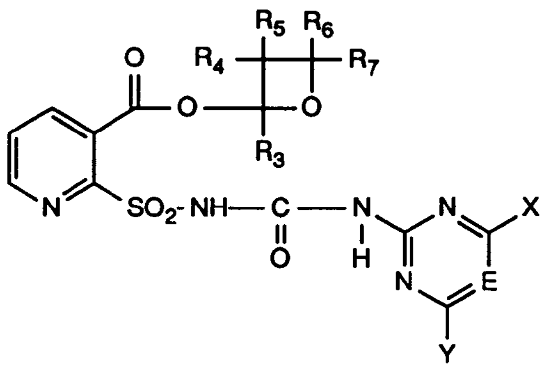

(Ia)

| Verb. Nr. | $R_3$ | $R_4$ | $R_5$ | $R_6$ | $R_7$ | X | Y | E | Schmp. [°C] |
|---|---|---|---|---|---|---|---|---|---|
| 1.001 | H | H | H | H | H | $OCH_3$ | $OCH_3$ | CH | 175-176 |
| 1.002 | H | H | H | H | H | $OCH_3$ | $CH_3$ | N | 157-158 |
| 1.003 | H | H | H | H | H | $OCH_3$ | $OCH_3$ | N | |
| 1.004 | H | H | H | H | H | $OCH_3$ | $CH_3$ | CH | 140-142 |
| 1.005 | H | H | H | H | H | $CH_3$ | $CH_3$ | CH | 145-147 |
| 1.006 | H | H | H | H | H | $OCH_3$ | Cl | CH | |
| 1.007 | H | H | H | H | H | $OCH_3$ | $OCHF_3$ | CH | |
| 1.008 | H | H | H | H | H | $OCH_3$ | 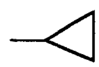 | N | |
| 1.009 | H | H | H | H | H | $OCH_2CF_3$ | $N(CH_3)_2$ | N | |
| 1.010 | $CH_3$ | H | H | H | H | $OCH_3$ | $OCH_3$ | CH | |
| 1.011 | $CH_3$ | H | H | H | H | $OCH_3$ | $CH_3$ | N | |
| 1.012 | $CH_3$ | H | H | H | H | $OCH_3$ | $OCH_3$ | N | |
| 1.013 | $CH_3$ | H | H | H | H | $OCH_3$ | $CH_3$ | CH | |
| 1.014 | $CH_3$ | H | H | H | H | $CH_3$ | $CH_3$ | CH | |
| 1.015 | $CH_3$ | H | H | H | H | $OCH_3$ | Cl | CH | |
| 1.016 | $CH_3$ | H | H | H | H | $OCH_3$ | $OCHF_2$ | CH | |
| 1.017 | $CH_3$ | H | H | H | H | $OCH_2CF_3$ | $N(CH_3)_2$ | N | |
| 1.018 | $CH_3$ | H | H | H | H | $OCH_3$ | 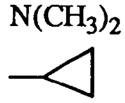 | N | |
| 1.019 | H | $CH_3$ | H | H | H | $OCH_3$ | $OCH_3$ | CH | |
| 1.020 | H | $CH_3$ | H | H | H | $OCH_3$ | $CH_3$ | N | |

| Verb. Nr. | $R_3$ | $R_4$ | $R_5$ | $R_6$ | $R_7$ | X | Y | E | Schmp. [°C] |
|---|---|---|---|---|---|---|---|---|---|
| 1.021 | H | $CH_3$ | H | H | H | $OCH_3$ | $OCH_3$ | N | |
| 1.022 | H | $CH_3$ | H | H | H | $OCH_3$ | $CH_3$ | CH | |
| 1.023 | H | $CH_3$ | H | H | H | $CH_3$ | $CH_3$ | CH | |
| 1.024 | H | $CH_3$ | H | H | H | $OCH_3$ | Cl | CH | |
| 1.025 | H | $CH_3$ | H | H | H | $OCH_3$ | $OCHF_2$ | CH | |
| 1.026 | H | $CH_3$ | H | H | H | $OCH_3$ | 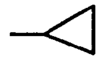 | N | |
| 1.027 | H | $CH_3$ | H | H | H | $OCH_2CF_3$ | $N(CH_3)_2$ | N | |
| 1.028 | H | $CH_3$ | $CH_3$ | H | H | $OCH_3$ | $OCH_3$ | CH | |
| 1.029 | H | $CH_3$ | $CH_3$ | H | H | $OCH_3$ | $CH_3$ | N | |
| 1.030 | H | $CH_3$ | $CH_3$ | H | H | $OCH_3$ | $OCH_3$ | N | |
| 1.031 | H | $CH_3$ | $CH_3$ | H | H | $OHC_3$ | $CH_3$ | CH | |
| 1.032 | H | $CH_3$ | $CH_3$ | H | H | $CH_3$ | $CH_3$ | CH | |
| 1.033 | H | $CH_3$ | $CH_3$ | H | H | $OCH_3$ | Cl | CH | |
| 1.034 | H | $CH_3$ | $CH_3$ | H | H | $OCH_3$ | $OCHF_2$ | CH | |
| 1.035 | H | $CH_3$ | $CH_3$ | H | H | $OCH_2CF_3$ | $N(CH_3)_2$ | N | |
| 1.036 | H | $CH_3$ | $CH_3$ | H | H | $OCH_3$ | 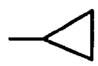 | N | |
| 1.037 | H | $CH_3$ | $CH_3$ | $CH_3$ | H | $OCH_3$ | $OCH_3$ | CH | |
| 1.038 | H | $CH_3$ | $CH_3$ | $CH_3$ | H | $OCH_3$ | $CH_3$ | N | |
| 1.039 | H | $CH_3$ | $CH_3$ | $CH_3$ | H | $OCH_3$ | $OCH_3$ | N | |
| 1.040 | H | $CH_3$ | $CH_3$ | $CH_3$ | H | $OCH_3$ | $CH_3$ | CH | |
| 1.041 | H | $CH_3$ | $CH_3$ | $CH_3$ | H | $CH_3$ | $CH_3$ | CH | |
| 1.042 | H | $CH_3$ | $CH_3$ | $CH_3$ | H | $OCH_3$ | Cl | CH | |
| 1.043 | H | $CH_3$ | $CH_3$ | $CH_3$ | H | $OCH_3$ | $OCHF_2$ | CH | |
| 1.044 | H | $CH_3$ | $CH_3$ | $CH_3$ | H | $OCH_2CF_3$ | $N(CH_3)_2$ | N | |
| 1.045 | H | $CH_3$ | $CH_3$ | $CH_3$ | H | $OCH_3$ | 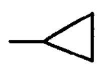 | N | |
| 1.046 | H | $CH_3$ | $CH_3$ | $CH_3$ | $CH_3$ | $OCH_3$ | $OCH_3$ | CH | |
| 1.047 | H | $CH_3$ | $CH_3$ | $CH_3$ | $CH_3$ | $OCH_3$ | $CH_3$ | N | |
| 1.048 | H | $CH_3$ | $CH_3$ | $CH_3$ | $CH_3$ | $OCH_3$ | $OCH_3$ | N | |
| 1.049 | H | $CH_3$ | $CH_3$ | $CH_3$ | $CH_3$ | $OCH_3$ | $CH_3$ | CH | |
| 1.050 | H | $CH_3$ | $CH_3$ | $CH_3$ | $CH_3$ | $CH_3$ | $CH_3$ | CH | |
| 1.051 | H | $CH_3$ | $CH_3$ | $CH_3$ | $CH_3$ | $OCH_3$ | Cl | CH | |
| 1.052 | H | $CH_3$ | $CH_3$ | $CH_3$ | $CH_3$ | $OCH_3$ | $OCHF_2$ | CH | |

| Verb. Nr. | $R_3$ | $R_4$ | $R_5$ | $R_6$ | $R_7$ | X | Y | E | Schmp. [°C] |
|---|---|---|---|---|---|---|---|---|---|
| 1.053 | H | $CH_3$ | $CH_3$ | $CH_3$ | $CH_3$ | $OCH_2CF_3$ | $N(CH_3)_2$ | N | |
| 1.054 | H | $CH_3$ | $CH_3$ | $CH_3$ | $CH_3$ | $OCH_3$ | 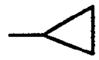 | N | |
| 1.055 | $CH_3$ | $CH_3$ | $CH_3$ | $CH_3$ | $CH_3$ | $OCH_3$ | $OCH_3$ | CH | |
| 1.056 | $CH_3$ | $CH_3$ | $CH_3$ | $CH_3$ | $CH_3$ | $OCH_3$ | $CH_3$ | N | |
| 1.057 | $CH_3$ | $CH_3$ | $CH_3$ | $CH_3$ | $CH_3$ | $OCH_3$ | $OCH_3$ | N | |
| 1.058 | $CH_3$ | $CH_3$ | $CH_3$ | $CH_3$ | $CH_3$ | $OCH_3$ | $CH_3$ | CH | |
| 1.059 | $CH_3$ | $CH_3$ | $CH_3$ | $CH_3$ | $CH_3$ | $CH_3$ | $CH_3$ | CH | |
| 1.060 | $CH_3$ | $CH_3$ | $CH_3$ | $CH_3$ | $CH_3$ | $OCH_3$ | Cl | CH | |
| 1.061 | $CH_3$ | $CH_3$ | $CH_3$ | $CH_3$ | $CH_3$ | $OCH_3$ | $OCHF_2$ | CH | |
| 1.062 | $CH_3$ | $CH_3$ | $CH_3$ | $CH_3$ | $CH_3$ | $OCH_2CF_3$ | $N(CH_3)_2$ | N | |
| 1.063 | $CH_3$ | $CH_3$ | $CH_3$ | $CH_3$ | $CH_3$ | $OCH_3$ | 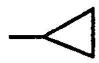 | N | |

Tabelle 2: Verbindung der Formel Ib

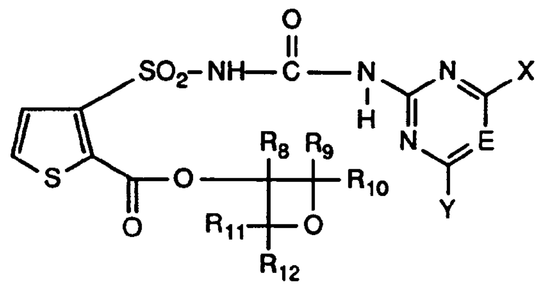

(Ib)

| Verb. Nr. | $R_8$ | $R_9$ | $R_{10}$ | $R_{11}$ | $R_{12}$ | X | Y | E | Schmp. [°C] |
|---|---|---|---|---|---|---|---|---|---|
| 2.001 | H | H | H | H | H | $OCH_3$ | $OCH_3$ | CH | 168-170 (Zers.) |
| 2.002 | H | H | H | H | H | $OCH_3$ | $CH_3$ | N | 126-128 |
| 2.003 | H | H | H | H | H | $OCH_3$ | $OCH_3$ | N | |
| 2.004 | H | H | H | H | H | $CH_3$ | $CH_3$ | CH | 183-185 |
| 2.005 | H | H | H | H | H | $OCH_3$ | Cl | CH | |
| 2.006 | H | H | H | H | H | $OCH_3$ | $OCHF_2$ | CH | |
| 2.007 | H | H | H | H | H | $OCH_3$ | 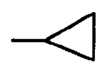 | N | |
| 2.008 | $CH_3$ | H | H | H | H | $OCH_3$ | $OCH_3$ | CH | |
| 2.009 | $CH_3$ | H | H | H | H | $OCH_3$ | $CH_3$ | N | |
| 2.010 | $CH_3$ | H | H | H | H | $OCH_3$ | $OCH_3$ | N | |
| 2.011 | $CH_3$ | H | H | H | H | $CH_3$ | $CH_3$ | CH | |
| 2.012 | $CH_3$ | H | H | H | H | $OCH_3$ | Cl | CH | |
| 2.013 | $CH_3$ | H | H | H | H | $OCH_3$ | $OCHF_2$ | CH | |
| 2.014 | $CH_3$ | H | H | H | H | $OCH_3$ | 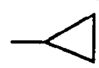 | N | |
| 2.015 | H | $CH_3$ | H | H | H | $OCH_3$ | $OCH_3$ | CH | |
| 2.016 | H | $CH_3$ | H | H | H | $OCH_3$ | $CH_3$ | N | |
| 2.017 | H | $CH_3$ | H | H | H | $OCH_3$ | $OCH_3$ | N | |
| 2.018 | H | $CH_3$ | H | H | H | $CH_3$ | $CH_3$ | CH | |
| 2.019 | H | $CH_3$ | H | H | H | $OCH_3$ | Cl | CH | |
| 2.020 | H | $CH_3$ | H | H | H | $OCH_3$ | $OCHF_2$ | CH | |
| 2.021 | H | $CH_3$ | H | H | H | $OCH_3$ | 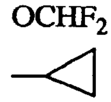 | N | |
| 2.022 | H | $CH_3$ | $CH_3$ | H | H | $OCH_3$ | $OCH_3$ | CH | |

| Verb. Nr. | $R_8$ | $R_9$ | $R_{10}$ | $R_{11}$ | $R_{12}$ | X | Y | E | Schmp. [°C] |
|---|---|---|---|---|---|---|---|---|---|
| 2.023 | H | $CH_3$ | $CH_3$ | H | H | $OCH_3$ | $CH_3$ | N | |
| 2.024 | H | $CH_3$ | $CH_3$ | H | H | $OCH_3$ | $OCH_3$ | N | |
| 2.025 | H | $CH_3$ | $CH_3$ | H | H | $CH_3$ | $CH_3$ | CH | |
| 2.026 | H | $CH_3$ | $CH_3$ | H | H | $OCH_3$ | Cl | CH | |
| 2.027 | H | $CH_3$ | $CH_3$ | H | H | $OCH_3$ | $OCHF_2$ | CH | |
| 2.028 | H | $CH_3$ | $CH_3$ | H | H | $OCH_3$ | 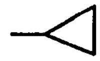 | N | |
| 2.029 | H | $CH_3$ | $CH_3$ | $CH_3$ | H | $OCH_3$ | $OCH_3$ | CH | |
| 2.030 | H | $CH_3$ | $CH_3$ | $CH_3$ | H | $OCH_3$ | $CH_3$ | N | |
| 2.031 | H | $CH_3$ | $CH_3$ | $CH_3$ | H | $OCH_3$ | $OCH_3$ | N | |
| 2.032 | H | $CH_3$ | $CH_3$ | $CH_3$ | H | $CH_3$ | $CH_3$ | CH | |
| 2.033 | H | $CH_3$ | $CH_3$ | $CH_3$ | H | $OCH_3$ | Cl | CH | |
| 2.034 | H | $CH_3$ | $CH_3$ | $CH_3$ | H | $OCH_3$ | $OCHF_2$ | CH | |
| 2.035 | H | $CH_3$ | $CH_3$ | $CH_3$ | H | $OCH_3$ | 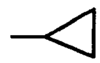 | N | |
| 2.036 | H | $CH_3$ | $CH_3$ | $CH_3$ | $CH_3$ | $OCH_3$ | $OCH_3$ | CH | |
| 2.037 | H | $CH_3$ | $CH_3$ | $CH_3$ | $CH_3$ | $OCH_3$ | $CH_3$ | N | |
| 2.038 | H | $CH_3$ | $CH_3$ | $CH_3$ | $CH_3$ | $OCH_3$ | $OCH_3$ | N | |
| 2.039 | H | $CH_3$ | $CH_3$ | $CH_3$ | $CH_3$ | $CH_3$ | $CH_3$ | CH | |
| 2.040 | H | $CH_3$ | $CH_3$ | $CH_3$ | $CH_3$ | $OCH_3$ | Cl | CH | |
| 2.041 | H | $CH_3$ | $CH_3$ | $CH_3$ | $CH_3$ | $OCH_3$ | $OCHF_2$ | CH | |
| 2.042 | H | $CH_3$ | $CH_3$ | $CH_3$ | $CH_3$ | $OCH_3$ | 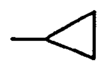 | N | |
| 2.043 | $CH_3$ | $CH_3$ | $CH_3$ | $CH_3$ | $CH_3$ | $OCH_3$ | $OCH_3$ | CH | |
| 2.044 | $CH_3$ | $CH_3$ | $CH_3$ | $CH_3$ | $CH_3$ | $OCH_3$ | $CH_3$ | N | |
| 2.045 | $CH_3$ | $CH_3$ | $CH_3$ | $CH_3$ | $CH_3$ | $OCH_3$ | $OCH_3$ | N | |
| 2.046 | $CH_3$ | $CH_3$ | $CH_3$ | $CH_3$ | $CH_3$ | $CH_3$ | $CH_3$ | CH | |
| 2.047 | $CH_3$ | $CH_3$ | $CH_3$ | $CH_3$ | $CH_3$ | $OCH_3$ | Cl | CH | |
| 2.048 | $CH_3$ | $CH_3$ | $CH_3$ | $CH_3$ | $CH_3$ | $OCH_3$ | $CCHF_2$ | CH | |
| 2.049 | $CH_3$ | $CH_3$ | $CH_3$ | $CH_3$ | $CH_3$ | $OCH_3$ | 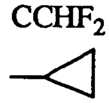 | N | |

Tabelle 3: Verbindung der Formel Ic

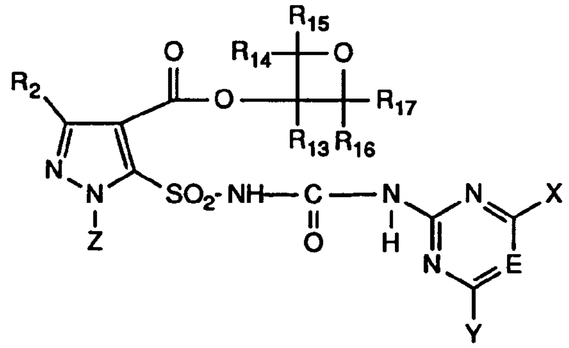

(Ic)

| Verb. Nr. | Z | $R_2$ | $R_{13}$ | $R_{14}$ | $R_{15}$ | $R_{16}$ | $R_{17}$ | X | Y | E | Schmp. [°C] |
|---|---|---|---|---|---|---|---|---|---|---|---|
| 3.001 | $CH_3$ | H | H | H | H | H | H | $OCH_3$ | $OCH_3$ | CH | 170-171 |
| 3.002 | $CH_3$ | H | H | H | H | H | H | $OCH_3$ | $CH_3$ | N | |
| 3.003 | $CH_3$ | H | H | H | H | H | H | $OCH_3$ | $OCH_3$ | N | |
| 3.004 | $CH_3$ | H | H | H | H | H | H | $CH_3$ | $CH_3$ | CH | |
| 3.005 | $CH_3$ | H | H | H | H | H | H | $OCH_3$ | Cl | CH | |
| 3.006 | $CH_3$ | H | H | H | H | H | H | $OCH_3$ | $OCHF_2$ | CH | |
| 3.007 | $CH_3$ | H | H | H | H | H | H | $OCH_3$ | 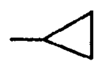 | N | |
| 3.008 | $CH_3$ | Cl | H | H | H | H | H | $OCH_3$ | $OCH_3$ | CH | |
| 3.009 | $CH_3$ | Cl | H | H | H | H | H | $OCH_3$ | $CH_3$ | N | |
| 3.010 | $CH_3$ | Cl | H | H | H | H | H | $OCH_3$ | $OCH_3$ | N | |
| 3.011 | $CH_3$ | Cl | H | H | H | H | H | $CH_3$ | $CH_3$ | CH | 169-170 |
| 3.012 | $CH_3$ | Cl | H | H | H | H | H | $OCH_3$ | Cl | CH | |
| 3.013 | $CH_3$ | Cl | H | H | H | H | H | $OCH_3$ | $OCHF_2$ | CH | |
| 3.014 | $CH_3$ | Cl | H | H | H | H | H | $OCH_3$ | 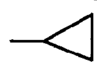 | N | |
| 3.015 | $CH_3$ | H | $CH_3$ | H | H | H | H | $OCH_3$ | $OCH_3$ | CH | |
| 3.016 | $CH_3$ | H | $CH_3$ | H | H | H | H | $OCH_3$ | $CH_3$ | N | |
| 3.017 | $CH_3$ | H | $CH_3$ | H | H | H | H | $OCH_3$ | $OCH_3$ | N | |
| 3.018 | $CH_3$ | H | $CH_3$ | H | H | H | H | $CH_3$ | $CH_3$ | H | |
| 3.019 | $CH_3$ | H | $CH_3$ | H | H | H | H | $OCH_3$ | Cl | CH | |

| Verb. Nr. | Z | $R_2$ | $R_{13}$ | $R_{14}$ | $R_{15}$ | $R_{16}$ | $R_{17}$ | X | Y | E | Schmp. [°C] |
|---|---|---|---|---|---|---|---|---|---|---|---|
| 3.020 | $CH_3$ | H | $CH_3$ | H | H | H | H | $OCH_3$ | $OCHF_2$ | CH | |
| 3.021 | $CH_3$ | H | $CH_3$ | H | H | H | H | $OCH_3$ | 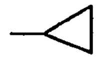 | N | |
| 3.022 | $CH_3$ | Cl | $CH_3$ | H | H | H | H | $OCH_3$ | $OCH_3$ | CH | |
| 3.023 | $CH_3$ | Cl | $CH_3$ | H | H | H | H | $OCH_3$ | $CH_3$ | N | |
| 3.024 | $CH_3$ | Cl | $CH_3$ | H | H | H | H | $OCH_3$ | $OCH_3$ | N | |
| 3.025 | $CH_3$ | Cl | $CH_3$ | H | H | H | H | $CH_3$ | $CH_3$ | CH | |
| 3.026 | $CH_3$ | Cl | $CH_3$ | H | H | H | H | $OCH_3$ | Cl | CH | |
| 3.027 | $CH_3$ | Cl | $CH_3$ | H | H | H | H | $OCH_3$ | $OCHF_2$ | CH | |
| 3.028 | $CH_3$ | Cl | $CH_3$ | H | H | H | H | $OCH_3$ | 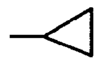 | N | |
| 3.029 | $CH_3$ | H | H | $CH_3$ | H | H | H | $OCH_3$ | $OCH_3$ | CH | |
| 3.030 | $CH_3$ | H | H | $CH_3$ | H | H | H | $OCH_3$ | $CH_3$ | N | |
| 3.031 | $CH_3$ | H | H | $CH_3$ | H | H | H | $OCH_3$ | $OCH_3$ | N | |
| 3.032 | $CH_3$ | H | H | $CH_3$ | H | H | H | $CH_3$ | $CH_3$ | CH | |
| 3.033 | $CH_3$ | H | H | $CH_3$ | H | H | H | $OCH_3$ | Cl | CH | |
| 3.034 | $CH_3$ | H | H | $CH_3$ | H | H | H | $OCH_3$ | $OCHF_2$ | CH | |
| 3.035 | $CH_3$ | H | H | $CH_3$ | H | H | H | $OCH_3$ | 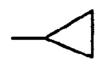 | N | |
| 3.036 | $CH_3$ | Cl | H | $CH_3$ | H | H | H | $OCH_3$ | $OCH_3$ | CH | |
| 3.037 | $CH_3$ | Cl | H | $CH_3$ | H | H | H | $OCH_3$ | $CH_3$ | N | |
| 3.038 | $CH_3$ | Cl | H | $CH_3$ | H | H | H | $OCH_3$ | $OCH_3$ | N | |
| 3.039 | $CH_3$ | Cl | H | $CH_3$ | H | H | H | $CH_3$ | $CH_3$ | CH | |
| 3.040 | $CH_3$ | Cl | H | $CH_3$ | H | H | H | $OCH_3$ | Cl | CH | |
| 3.041 | $CH_3$ | Cl | H | $CH_3$ | H | H | H | $OCH_3$ | $CCHF_2$ | CH | |
| 3.042 | $CH_3$ | Cl | H | $CH_3$ | H | H | H | $OCH_3$ | 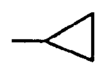 | N | |
| 3.043 | 2-Pyr. | H | H | H | H | H | H | $OCH_3$ | $OCH_3$ | CH | 165-166 |
| 3.044 | 2-Pyr. | H | H | H | H | H | H | $OCH_3$ | $CH_3$ | N | 159-162 |
| 3.045 | 2-Pyr. | H | H | H | H | H | H | $OCH_3$ | $OCH_3$ | N | |
| 3.046 | 2-Pyr. | H | H | H | H | H | H | $CH_3$ | $CH_3$ | CH | 177-179 |
| 3.047 | 2-Pyr. | H | H | H | H | H | H | $OCH_3$ | Cl | CH | |
| 3.048 | 2-Pyr. | H | H | H | H | H | H | $OCH_3$ | $OCHF_2$ | CH | |
| 3.049 | 2-Pyr. | H | H | H | H | H | H | $OCH_3$ | 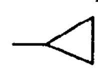 | N | |

| Verb. Nr. | Z | $R_2$ | $R_{13}$ | $R_{14}$ | $R_{15}$ | $R_{16}$ | $R_{17}$ | X | Y | E | Schmp. [°C] |
|---|---|---|---|---|---|---|---|---|---|---|---|
| 3.050 | 2-Pyr. | H | H | H | H | H | H | $OCH_3$ | $CH_3$ | CH | 169-170 |
| 3.051 | 2-Pyr. | Cl | H | H | H | H | H | $OCH_3$ | $OCH_3$ | CH | |
| 3.052 | 2-Pyr. | Cl | H | H | H | H | H | $OCH_3$ | $CH_3$ | N | |
| 3.053 | 2-Pyr. | Cl | H | H | H | H | H | $OCH_3$ | $OCH_3$ | N | |
| 3.054 | 2-Pyr. | Cl | H | H | H | H | H | $CH_3$ | $CH_3$ | CH | |
| 3.055 | 2-Pyr. | Cl | H | H | H | H | H | $OCH_3$ | Cl | CH | |
| 3.056 | 2-Pyr. | Cl | H | H | H | H | H | $OCH_3$ | $OCHF_2$ | CH | |
| 3.057 | 2-Pyr. | Cl | H | H | H | H | H | $OCH_3$ | 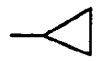 | N | |
| 3.058 | 2-Pyr. | H | $CH_3$ | H | H | H | H | $OCH_3$ | $OCH_3$ | CH | |
| 3.059 | 2-Pyr. | H | $CH_3$ | H | H | H | H | $OCH_3$ | $CH_3$ | N | |
| 3.060 | 2-Pyr. | H | $CH_3$ | H | H | H | H | $OCH_3$ | $OCH_3$ | N | |
| 3.061 | 2-Pyr. | H | $CH_3$ | H | H | H | H | $CH_3$ | $CH_3$ | CH | |
| 3.062 | 2-Pyr. | H | $CH_3$ | H | H | H | H | $OCH_3$ | Cl | CH | |
| 3.063 | 2-Pyr. | H | $CH_3$ | H | H | H | H | $OCH_3$ | $OCHF_2$ | CH | |
| 3.064 | 2-Pyr. | H | $CH_3$ | H | H | H | H | $OCH_3$ | 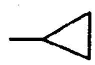 | N | |
| 3.065 | 2-Pyr. | Cl | $CH_3$ | H | H | H | H | $OCH_3$ | $OCH_3$ | CH | |
| 3.066 | 2-Pyr. | Cl | $CH_3$ | H | H | H | H | $OCH_3$ | $CH_3$ | N | |
| 3.067 | 2-Pyr. | Cl | $CH_3$ | H | H | H | H | $OCH_3$ | $OCH_3$ | N | |
| 3.068 | 2-Pyr. | Cl | $CH_3$ | H | H | H | H | $CH_3$ | $CH_3$ | CH | |
| 3.069 | 2-Pyr. | Cl | $CH_3$ | H | H | H | H | $OCH_3$ | Cl | CH | |
| 3.070 | 2-Pyr. | Cl | $CH_3$ | H | H | H | H | $OCH_3$ | $OCHF_2$ | CH | |
| 3.071 | 2-Pyr. | H | $CH_3$ | $CH_3$ | H | H | H | $OCH_3$ | 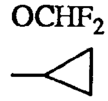 | N | |
| 3.072 | 2-Pyr. | H | $CH_3$ | $CH_3$ | H | H | H | $OCH_3$ | $OCH_3$ | CH | |
| 3.073 | 2-Pyr. | H | $CH_3$ | $CH_3$ | H | H | H | $OCH_3$ | $CH_3$ | N | |
| 3.074 | 2-Pyr. | H | $CH_3$ | $CH_3$ | H | H | H | $OCH_3$ | $OCH_3$ | N | |
| 3.075 | 2-Pyr. | H | $CH_3$ | $CH_3$ | H | H | H | $CH_3$ | $CH_3$ | CH | |
| 3.076 | 2-Pyr. | H | $CH_3$ | $CH_3$ | H | H | H | $OCH_3$ | Cl | CH | |
| 3.077 | 2-Pyr. | H | $CH_3$ | $CH_3$ | H | H | H | $OCH_3$ | $OCHF_2$ | CH | |
| 3.078 | 2-Pyr. | H | $CH_3$ | $CH_3$ | H | H | H | $OCH_3$ | 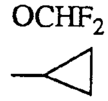 | N | |
| 3.079 | 2-Pyr. | Cl | $CH_3$ | $CH_3$ | H | H | H | $OCH_3$ | $OCH_3$ | CH | |

| Verb. Nr. | Z | $R_2$ | $R_{13}$ | $R_{14}$ | $R_{15}$ | $R_{16}$ | $R_{17}$ | X | Y | E | Schmp. [°C] |
|---|---|---|---|---|---|---|---|---|---|---|---|
| 3.080 | 2-Pyr. | Cl | $CH_3$ | $CH_3$ | H | H | H | $OCH_3$ | $CH_3$ | N | |
| 3.081 | 2-Pyr. | Cl | $CH_3$ | $CH_3$ | H | H | H | $OCH_3$ | $OCH_3$ | N | |
| 3.082 | 2-Pyr. | Cl | $CH_3$ | $CH_3$ | H | H | H | $CH_3$ | $CH_3$ | CH | |
| 3.083 | 2-Pyr. | Cl | $CH_3$ | $CH_3$ | H | H | H | $OCH_3$ | Cl | CH | |
| 3.084 | 2-Pyr. | Cl | $CH_3$ | $CH_3$ | H | H | H | $OCH_3$ | $OCHF_2$ | CH | |
| 3.085 | 2-Pyr. | Cl | $CH_3$ | $CH_3$ | H | H | H | $OCH_3$ | 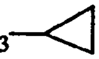 | N | |

Tabelle 4: Verbindungen der Formel IIa

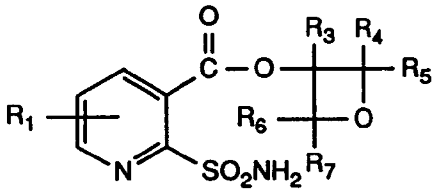

(IIa)

| Verb. Nr. | $R_1$ | $R_3$ | $R_4$ | $R_5$ | $R_6$ | $R_7$ | Schmp. [°C] |
|---|---|---|---|---|---|---|---|
| 4.001 | H | H | H | H | H | H | 142-144 |
| 4.002 | H | $CH_3$ | H | H | H | H | |
| 4.003 | H | H | $CH_3$ | H | H | H | |
| 4.004 | H | H | $CH_3$ | $CH_3$ | H | H | |
| 4.005 | H | H | $CH_3$ | $CH_3$ | $CH_3$ | H | |
| 4.006 | H | H | $CH_3$ | $CH_3$ | $CH_3$ | $CH_3$ | |
| 4.007 | H | $CH_3$ | $CH_3$ | $CH_3$ | $CH_3$ | $CH_3$ | |

Tabelle 5: Verbindungen der Formel IIb

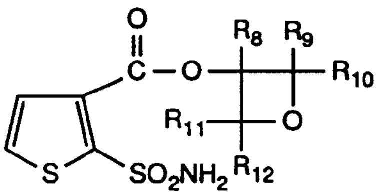

(IIb)

| Verb. Nr. | $R_8$ | $R_9$ | $R_{10}$ | $R_{11}$ | $R_{12}$ | Schmp. [°C] |
|---|---|---|---|---|---|---|
| 5.001 | H | H | H | H | H | 158-160 |
| 5.002 | $CH_3$ | H | H | H | H | |
| 5.003 | H | $CH_3$ | H | H | H | |
| 5.004 | H | $CH_3$ | $CH_3$ | H | H | |
| 5.005 | H | $CH_3$ | $CH_3$ | $CH_3$ | H | |
| 5.006 | H | $CH_3$ | $CH_3$ | $CH_3$ | $CH_3$ | |
| 5.007 | $CH_3$ | $CH_3$ | $CH_3$ | $CH_3$ | $CH_3$ | |

Tabelle 6: Verbindungen der Formel IIc

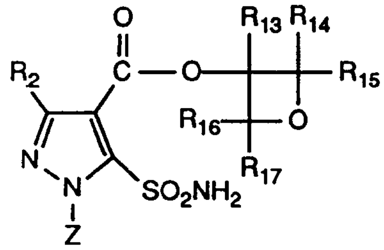

(IIc)

| Verb. Nr. | Z | $R_2$ | $R_{13}$ | $R_{14}$ | $R_{15}$ | $R_{16}$ | $R_{17}$ | Schmp. [°C] |
|---|---|---|---|---|---|---|---|---|
| 6.001 | $CH_3$ | H | H | H | H | H | H | 145-147 |
| 6.002 | $CH_3$ | Cl | H | H | H | H | H | |
| 6.003 | $CH_3$ | H | $CH_3$ | H | H | H | H | |
| 6.004 | $CH_3$ | H | H | $CH_3$ | H | H | H | |
| 6.005 | $CH_3$ | H | H | $CH_3$ | $CH_3$ | H | H | |
| 6.006 | $CH_3$ | H | H | $CH_3$ | $CH_3$ | $CH_3$ | H | |
| 6.007 | $CH_3$ | H | H | $CH_3$ | $CH_3$ | $CH_3$ | $CH_3$ | |
| 6.008 | $CH_3$ | H | $CH_3$ | $CH_3$ | $CH_3$ | $CH_3$ | $CH_3$ | |
| 6.009 | $CH_3$ | H | H | H | H | H | $C_2H_5$ | |
| 6.010 | $CH_3$ | Cl | $CH_3$ | H | H | H | H | |
| 6.011 | $CH_3$ | Cl | H | $CH_3$ | H | H | H | |
| 6.012 | $CH_3$ | Cl | H | $CH_3$ | $CH_3$ | H | H | |
| 6.013 | $CH_3$ | Cl | H | $CH_3$ | $CH_3$ | $CH_3$ | H | |
| 6.014 | $CH_3$ | Cl | H | $CH_3$ | $CH_3$ | $CH_3$ | $CH_3$ | |
| 6.015 | $CH_3$ | Cl | $CH_3$ | $CH_3$ | $CH_3$ | $CH_3$ | $CH_3$ | |
| 6.016 | 2-Pyr. | H | H | H | H | H | H | |
| 6.017 | 2-Pyr. | Cl | H | H | H | H | H | |
| 6.018 | 2-Pyr. | H | $CH_3$ | H | H | H | H | |
| 6.019 | 2-Pyr. | H | H | $CH_3$ | H | H | H | |
| 6.020 | 2-Pyr. | H | H | $CH_3$ | $CH_3$ | H | H | |
| 6.021 | 2-Pyr. | H | H | $CH_3$ | $CH_3$ | $CH_3$ | H | |
| 6.022 | 2-Pyr. | H | H | $CH_3$ | $CH_3$ | $CH_3$ | $CH_3$ | |
| 6.023 | 2-Pyr. | H | $CH_3$ | $CH_3$ | $CH_3$ | $CH_3$ | $CH_3$ | |
| 6.024 | 2-Pyr. | Cl | $CH_3$ | H | H | H | H | |

| Verb. Nr. | Z | $R_2$ | $R_{13}$ | $R_{14}$ | $R_{15}$ | $R_{16}$ | $R_{17}$ | Schmp. [°C] |
|---|---|---|---|---|---|---|---|---|
| 6.025 | 2-Pyr. | Cl | H | $CH_3$ | | H | H | |
| 6.026 | 2-Pyr. | Cl | H | $CH_3$ | $CH_3$ | H | H | |
| 6.027 | 2-Pyr. | Cl | H | $CH_3$ | $CH_3$ | $CH_3$ | H | |
| 6.028 | 2-Pyr. | Cl | H | $CH_3$ | $CH_3$ | $CH_3$ | $CH_3$ | |
| 6.029 | 2-Pyr. | Cl | $CH_3$ | $CH_3$ | $CH_3$ | $CH_3$ | $CH_3$ | |

Formulierungsbeispiele für Wirkstoffe der Formel I (% = Gewichtsprozent)

| F1. Spritzpulver | a) | b) | c) |
|---|---|---|---|
| Wirkstoff gemäß Tabellen 1-3 | 20 % | 50 % | 0,5 % |
| Na-Ligninsulfonat | 5 % | 5 % | 5 |
| Na-Laurylsulfat | 3 % | - | - % |
| Na-Diisobutylnaphthalinsulfonat | - | 6 % | 6 % |
| Octylphenolpolyethylenglykolether (7-8 Mol AeO) | - | 2 % | 2 |
| Hochdisperse Kieselsäure | 5 % | 27% | 27 % |
| Kaolin | 67 % | - % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F2. Wasserdispergierbares Granulat | a) | b) |
|---|---|---|
| Wirkstoff gemäß Tabellen 1-3 | 75% | 5 % |
| Natrium-dibutylnaphtalinsulfonat | 2 % | 0,5 % |
| Gummi arabicum | 1 % | 1 % |
| Natriumsulfat | 5 % | 3% |
| Natriumligninsulfonat | 17% | 15 % |
| Kaolin | - | 75,5 % |

Aus solchen Konzentraten können durch Verdünnen mit Wasser Emulsionen jeder gewünschten Konzentration hergestellt werden.

| F3. Stäubemittel | a) | b) |
|---|---|---|
| Wirkstoff gemäß Tabellen 1-3 | 0,1 % | 1 % |
| Talkum | 99,9 % | - |
| Kaolin | - | 99 % |

Durch inniges Vermischen der Trägerstoffe mit dem Wirkstoff erhält man gebrauchsfertige Stäubemittel.

| F4. Extruder-Granulat | a) | b) |
|---|---|---|
| Wirkstoff gemäß Tabellen 1-3 | 10 % | 1 % |
| Na-Ligninsulfonat | 2 % | 2 % |
| Carboxymethylcellulose | 1 % | 1 % |
| Kaolin | 87 % | 96 % |

Der Wirkstoff mit den Zusatzstoffen vermischt, vermahlen und mit Wasser angefeuchtet. Dieses Gemisch wird extrudiert und anschließend im Luftstrom getrocknet.

| F5. Umhüllungs-Granulat | |
|---|---|
| Wirkstoff gemäß Tabellen 1-3 | 3 % |
| Polyäthylenglykol (MG200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| F6. Suspensions-Konzentrat | a) | b) |
|---|---|---|
| Wirkstoff gemäß Tabellen 1-3 | 5 % | 40 % |
| Ethylenglykol | 10 % | 10 % |
| Nonylphenolpolyethylenglykolether (15 Mol AeO) | 1 % | 6 % |
| Na-Ligninsulfonat | 5 % | 10 % |
| Carboxymethylcellulose | 1 % | 1 % |
| 37 %ige wäßrige Formaldehyd-Lösung | 0,2 % | 0,2 % |
| Silikonöl in Form einer 75%-igen wäßrigen Emulsion | 0,8 % | 0,8 % |
| Wasser | 77 % | 32 % |

Der fein gemahlene Wirkstoff wird mit den Zusatzstoffen innig vermischt. Man erhält so ein Suspensionskonzentrat, aus welchem durch Verdünnen mit Wasser Suspensionen jeder gewünschten Konzentration hergestellt werden können.

| F7. Salzlösung | |
|---|---|
| Wirkstoff gemäß Tabellen 1-3 | 5 % |
| Isopropylamin | 1 % |
| Octylphenolpolyethylenglykolether (78 Mol AeO) | 3 % |
| Wasser | 91 % |

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Biologische Beispiele

Beispiel B1: Herbizidwirkung vor dem Auflaufen der Pflanzen

Kunststofftöpfe werden mit expandiertem Vermiculit (Dichte: 0,135 g/cm$^3$, Wasseradsorptionsvermögen: 0,565 l/l) gefüllt. Nach dem Sättigen des nicht adsorptiven Vermiculits mit einer wäßrigen Wirkstoffemulsion in deionisiertem Wasser, die die Wirkstoffe in einer Konzentration von 70 ppm enthält, werden Samen der folgenden Pflanzen auf die Oberfläche gesät: Nasturtium officinalis, Agrostis tenuis, Stellaria media und Digitaria sanguinalis. Die Versuchsgefäße werden anschließend in einer Klimakammer bei einer Temperatur von 20 °C, einer Beleuchtung von ca. 20 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Gießwasser 0,5 % eines handelsüblichen Flüssigdüngers zugesetzt. 12 Tage nach der Aussaat wird der Versuch ausgewertet und die Wirkung auf die Versuchspflanzen nach dem folgenden Maßstab bewertet:

1 :Pflanze nicht gekeimt oder total abgestorben
2-3 :sehr starke Wirkung
4-6 :mittlere Wirkung
7-8 :schwache Wirkung
9 :keine Wirkung (wie unbehandelte Kontrolle)

Tabelle B1: preemergente Wirkung:

Konzentration der Wirkstoffemulsion: 70 ppm

| Testpflanze: Wirkstoff Nr. | Nasturtium | Stellaria | Agrostis | Digitaria |
|---|---|---|---|---|
| 1.001 | 2 | 2 | 2 | 2 |
| 1.002 | 2 | 2 | 3 | 3 |
| 1.004 | 5 | 4 | 3 | 4 |
| 1.005 | 3 | 3 | 3 | 3 |
| 2.001 | 2 | 2 | 1 | 2 |
| 2.002 | 2 | 2 | 4 | 2 |
| 2.004 | 3 | 2 | 2 | 2 |

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wäßrigen Wirktoffdispersion gemäß Beispiel F6 in einer Dosierung von 8-500 g Wirksubstanz pro Hektar auf die Pflanzen gespritzt und diese bei 24°-26°C und 45-60 % relativer Luftfeuchtigkeit gehalten. 15 Tage nach der Behandlung wird der Versuch ausgewertet.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet. Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Formel I starke Herbizidwirkung. Dieselben Resultate werden erhalten, wenn man die Verbindungen der Formel I gemäß den Beispielen F1 bis F5 und F7 formuliert.

**Patentansprüche**

1. Verbindungen Formel I

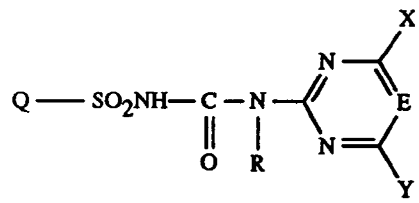

(I)

worin

Q für

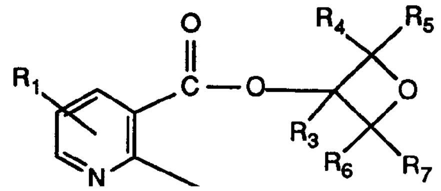

($Q_1$),

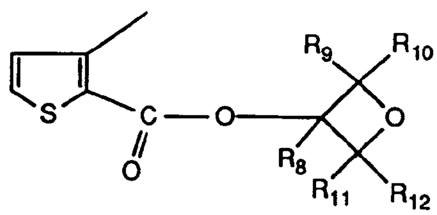

($Q_2$)

oder

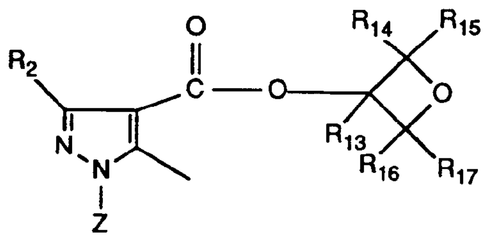

($Q_3$);

| | |
|---|---|
| R | für Wasserstoff oder Methyl; |
| $R_1$ | für Wasserstoff, Fluor, Chlor, $C_1$-$C_4$-Alkyl oder Methoxy; |
| $R_2$ | für Wasserstoff, Fluor oder Chlor; |
| $R_2$ | für Wasserstoff, Fluor oder Chlor; |
| $R_3$, $R_4$, $R_5$, $R_6$ und $R_7$ | unabhängig voneinander für Wasserstoff oder $C_1$-$C_4$-Alkyl; |
| $R_8$, $R_9$, $R_{10}$, $R_{11}$ und $R_{12}$ | unabhängig voneinander für Wasserstoff oder $C_1$-$C_4$-Alkyl; |
| $R_{13}$, $R_{14}$, $R_{15}$, $R_{16}$ und $R_{17}$ | unabhängig voneinander für Wasserstoff oder $C_1$-$C_4$-Alkyl; |
| Z | für Methyl oder 2-Pyridyl; |
| E | für Methin oder Stickstoff; |
| X | für $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, $C_2$-$C_5$-Alkoxyalkyl, $C_2$-$C_5$-Alkoxyalkoxy, $C_2$-$C_{54}$-Alkylthioalkyl oder Cyclopropyl; |
| Y | für $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino oder Di-($C_1$-$C_3$Alkyl)amino stehen; |

sowie die Salze dieser Verbindungen.

2. Verbindungen der Formel I gemäß Anspruch 1, dadurch gekennzeichnet, daß R Wasserstoff bedeutet.

3. Verbindungen der Formel I gemäß Anspruch 2, dadurch gekennzeichnet, daß E für Methin steht.

4. Verbindungen der Formel I gemäß Anspruch 2, dadurch gekennzeichnet, daß E für Stickstoff steht.

5. Verbindungen der Formel I gemäß einem der Ansprüche 3 und 4, dadurch gekennzeichnet, daß Q für $Q_1$ steht.

6. Verbindungen der Formel I gemäß einem der Ansprüche 3 und 4, dadurch gekennzeichnet, daß Q für $Q_2$, X für $C_1$-$C_3$-Alkyl, $C_1$-$C_3$-Alkoxy, $C_1$-$C_2$halogenalkoxy oder Cyclopropyl; und Y für $C_1$ -$C_3$-Alkyl, $C_1$-$C_3$-Alkoxy, $C_1$-$C_2$Halogenalkoxy, Trifluormethyl, Difluormethyl, Flurомethyl, Methoxymethyl, Fluor, Chlor, Amino, Methylamino, Dimethylamino, oder Methylthio stehen.

7. Verbindungen der Formel I gemäß einem der Ansprüche 3 und 4, dadurch gekennzeichnet, daß Q für $Q_3$ steht.

8. Verbindungen der Formel I gemäß Anspruch 5, dadurch gekennzeichnet, daß $R_1$ Wasserstoff bedeutet.

9. Verbindungen der Formel I gemäß Anspruch 7, dadurch gekennzeichnet, daß $R_2$ Wasserstoff oder Chlor bedeutet.

10. Verbindungen der Formel I gemäß Anspruch 5, dadurch gekennzeichnet, daß $R_3$, $R_4$, $R_5$, $R_6$ und $R_7$ Wasserstoff bedeuten.

11. Verbindungen der Formel I gemäß Anspruch 6, dadurch gekennzeichnet, daß $R_8$, $R_9$, $R_{10}$, $R_{11}$ und $R_{12}$ Wasserstoff bedeuten.

12. Verbindungen der Formel I gemäß Anspruch 7, dadurch gekennzeichnet, daß $R_{13}$, $R_{14}$, $R_{15}$, $R_{16}$ und $R_{17}$ Wasserstoff bedeuten.

13. Verbindungen der Formel I gemäß Anspruch 1, dadurch gekennzeichnet, daß X für $C_1$-$C_3$-Alkyl, $C_1$-$C_3$-Alkoxy, $C_1$-$C_2$Halogenalkoxy oder Cyclopropyl; und Y für $C_1$-$C_3$-Alkyl, $C_1$-$C_3$-Alkoxy, $C_1$-$C_2$-Halogenalkoxy, Trifluormethyl, Difluormethyl, Flurometyl, Methoxymethyl, Fluor, Chlor, Amino, Methylamino, Dimethylamino, oder Methylthio stehen.

14. Verbindungen der Formel I gemäß Anspruch 13, dadurch gekennzeichnet, daß X für Methyl, Methoxy, Difluormethoxy, Ethoxy oder Cyclopropyl; und Y für Methyl, Ethyl, Methoxy, Difluormethoxy, Ethoxy, 2,2,2,-Trifluorethoxy, Chlor, Methylamino, Dimethylamino oder Methoxymethyl stehen.

15. Verfahren zur Herstellung der Verbindungen der Formel I, dadurch gekennzeichnet, daß man entweder
a) ein Sulfonamid der Formeln IIa IIb oder IIc

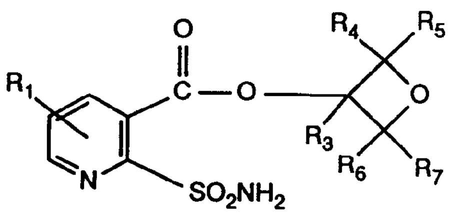

(IIa),

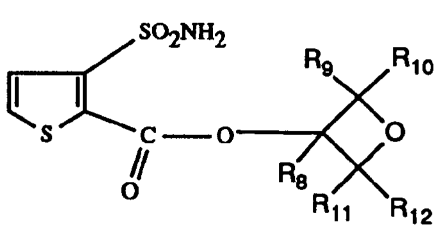

(IIb),

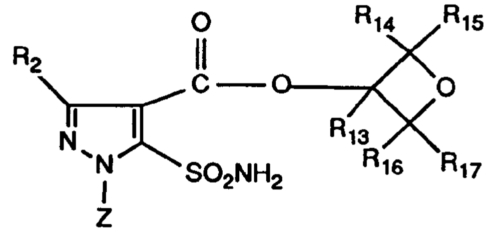

(IIc),

worin die Substituenten jeweils die unter Formel I in Anspruch 1 angegebenen Bedeutungen haben, in Gegenwart einer Base mit einem Pyrimidinyl-, oder Triazinylcarbamat der Formel III

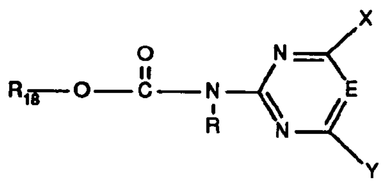

(III),

worin R, X und Y die unter Formel I in Anspruch 1 angegebenen Bedeutungen haben und $R_{18}$ für Phenyl oder durch $C_1$-$C_4$-Alkyl oder Halogen substituiertes Phenyl steht, umsetzt, oder
b) ein Sulfonylcarbamat der Formel IVa IVb, oder IVc

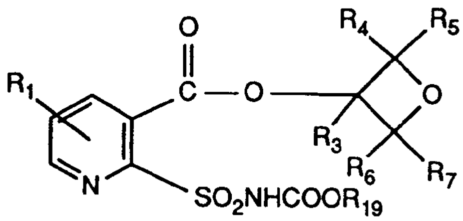

(IVa),

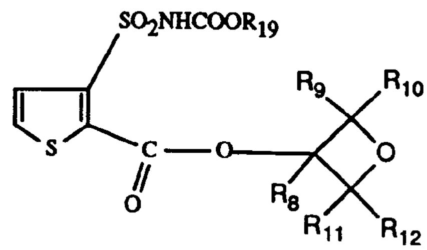

(IVb),

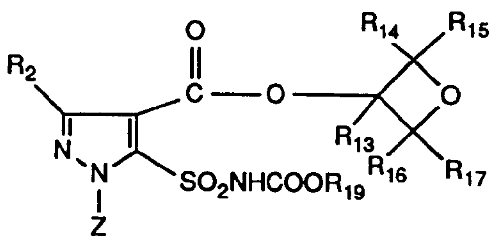

(IVc),

worin die Substituenten jeweils die unter Formel I in Anspruch 1 angegebenen Bedeutungen haben und $R_{19}$ für Phenyl oder durch $C_1$-$C_4$-Alkyl oder Halogen substituiertes Phenyl steht, in Gegenwart einer Base mit einem Amin der Formel V

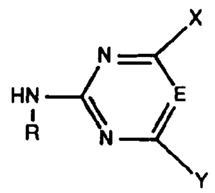

(V)

worin R, E, X und Y die unter Formel I in Anspruch 1 angegebenen Bedeutungen haben, umsetzt, oder c) ein Sulfonamid der Formeln IIa, IIb oder IIc

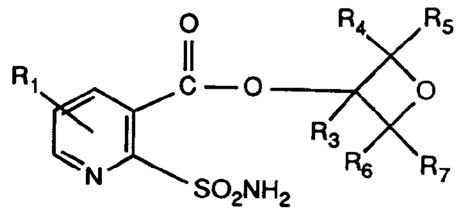

(IIa),

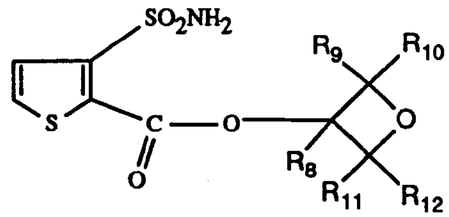

(IIb),

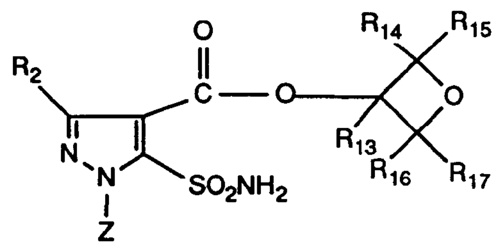

(IIc),

worin die Substituenten jeweils die unter Formel I in Anspruch 1 angegebenen Bedeutungen haben, in Gegenwart einer Base mit einem Pyrimidinyl- oder Triazinylisocyanat der Formel VI

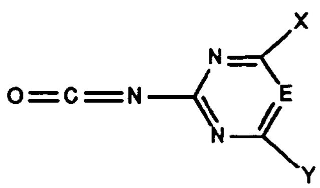

(VI)

worin E, X und Y die unter Formel I in Anspruch 1 angegebenen Bedeutungen haben, umsetzt.

**16.** Sulfonamide der Formel IIa

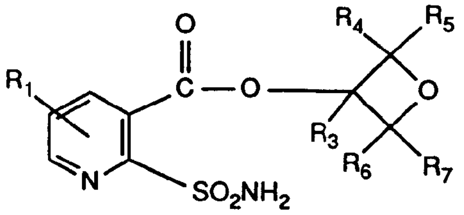

(IIa),

worin die Substituenten $R_1$, $R_3$, $R_4$, $R_5$, $R_6$ und $R_7$ die unter Formel I in Anspruch 1 angegebene Bedeutung haben.

**17.** Sulfonamide der Formel IIb

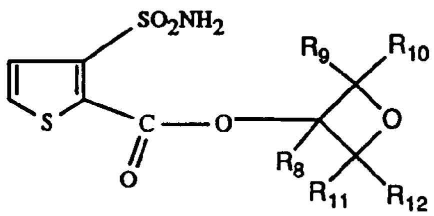

(IIb),

worin die Substituenten $R_5$, $R_9$, $R_{10}$, $R_{11}$ und $R_{12}$ die unter Formel I in Anspruch 1 angegebene Bedeutung haben.

**18.** Sulfonamide der Formel IIc

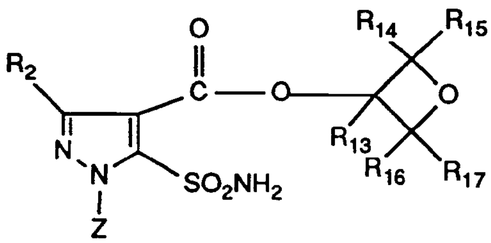

(IIc),

worin die Substituenten $R_2$, $R_{13}$, $R_{14}$, $R_{15}$, $R_{16}$ und $R_{17}$ die unter Formel I in Anspruch 1 angegebene Bedeutung haben.

**19.** Ein herbizides und den Pflanzenwuchs hemmendes Mittel, gekennzeichnet durch einen Gehalt an einem oder mehreren Sulfonylharnstoffen der Formel I, gemäß Anspruch 1.

**20.** Mittel gemäß Anspruch 19, dadurch gekennzeichnet, daß es zwischen 0,1 % und 95 % Wirkstoff der Formel I gemäß Anspruch 1 enthält.

**21.** Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pfanzen oder deren Lebensraum appliziert.

**22.** Verfahren gemäß Anspruch 21, dadurch gekennzeichnet, daß man eine Wirkstoffmenge zwischen 0,01 und 2 kg pro Hektar appliziert.

**23.** Verfahren zur Hemmung des Pflanzenwachstums, dadurch gekennzeichnet, daß man einen Wirkstoff der Formel I, gemaß Anspruch 1, oder ein diesen Wirkstoff enthaltendes Mittel in einer wirksamen Menge auf die Pflanzen oder deren Lebensraum appliziert.

**24.** Verfahren gemäß Anspruch 21 zur selektiven pre- oder post-emergenten Bekämpfung von Unkräutem in Nutzpflanzenkulturen.

25. Verwendung eines Mittels gemäß Anspruch 19 zur selektiven pre- oder post-emergenten Bekämpfung von Unkräutern in Nutzpflanzenkulturen.

Europäisches Patentamt

# EUROPÄISCHER RECHERCHENBERICHT

Nummer der Anmeldung

EP 93 81 0085

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D | EP-A-0 007 687 (E.I. DU PONT DE NEMOURS AND COMPANY)<br>* Seite 34, Zeile 25; Ansprüche 1,9-11,14 *<br>--- | 1-25 | C07D405/14<br>C07D409/14<br>C07D405/12<br>C07D409/12<br>A01N47/36 |
| A | US-A-4 892 946 (G. LEVITT)<br>* Anspruch 5; Beispiel 3; Tabellen * | 1-25 | |
| D | & EP-A-0 030 138 (E.I. DU PONT DE NEMOURS AND COMPANY)<br>----- | | |

| RECHERCHIERTE SACHGEBIETE (Int. Cl.5 ) |
|---|
| C07D |

Der vorliegende Recherchenbericht wurde für alle Patentansprüche erstellt

| Recherchenort | Abschlußdatum der Recherche | Prüfer |
|---|---|---|
| DEN HAAG | 13 APRIL 1993 | P. BOSMA |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)